# EUROPEAN PATENT APPLICATION

(11) **EP 4 063 496 A1**
(43) Date of publication of application: **28.09.2022**
(21) Application number: 20890883.0
(22) Date of filing: 19.11.2020
(51) Int. Cl.: C12N 5/10, A61K 35/545, A61P 25/16, A61P 43/00, C12N 1/04, C12N 5/0793

(54) **METHOD FOR FREEZING NEURAL CELLS**

(30) Priority: 20.11.2019 JP 2019209929
(71) Applicant: Sumitomo Pharma Co., Ltd., Osaka 541-0045 (JP); Kyoto University, Kyoto-shi, Kyoto 606-8501 (JP)
(72) Inventor: HIRAMATSU, Satoe, Kobe-shi, Hyogo 650-0047 (JP); NAKAGAWA, Takashi, Suita-shi, Osaka 564-0053 (JP); YOSHIDA, Kenji, Kobe-shi, Hyogo 650-0047 (JP); TAKAHASHI, Jun, Kyoto-shi, Kyoto 606-8501 (JP); DOI, Daisuke, Kyoto-shi, Kyoto 606-8501 (JP)
(74) Representative: Mewburn Ellis LLP
(86) International application number: PCT/JP2020/043275
(87) International publication number: WO 2021/100829

(57) **Abstract**

Provided is a method for freezing a cell aggregate including neural cells. provided is a method for freezing a cell aggregate including neural cells and having a three-dimensional structure, which comprises following steps (1) and (2): (1) contacting a cell aggregate including neural cells and having a three-dimensional structure with a preservation solution at 0°C to 30°C prior to freezing to prepare a preservation solution-soaked cell aggregate; and (2) cooling the preservation solution-soaked cell aggregate obtained in step (1) from a temperature at least about 5°C higher than the freezing point of the preservation solution to a temperature about 5°C lower than the freezing point at an average cooling speed of 2 to 7 °C/min to freeze the cell aggregate.

## Description

### TECHNICAL FIELD

The present application relates to a method for freezing cell aggregates including neural cells.

### BACKGROUND

Transplantation of dopamine-producing (DA) neurons has been considered as a promising therapeutic method for Parkinson's disease (PD) based on reports on clinical trials using fetal mesencephalic cells (Non Patent Literature 1). In addition, a method for inducing differentiation of pluripotent stem cells (PSCs) such as embryonic stem cells (ES cells or ESCs) or induced pluripotent stem cells (iPS cells or iPSCs) into dopamine-producing neurons, or progenitor cells thereof has been reported. The group of the present inventors has already reported a method for preparing dopamine-producing neurons, or dopamine-producing neuron progenitor cells from human induced pluripotent stem cells (Non Patent Literature 2). Other groups have also reported preparation of PSC-derived dopamine-producing nerves (Non Patent Literatures 3 and 4) .

For drugs having cells as active ingredients, cryopreservation of a final product is an essential element for the dissemination of cell therapies (Patent Literatures 1 to 4). Unlike cell biology research, it is preferable that cryopreserved cells, when used in clinical practice, be transplanted immediately after thawing without recovery culture. Therefore, it is important for frozen cells to maintain their engraftment capacity, function or activity, and cell viability after thawing.

It has been suggested that transplantation of solid tissue induces a stronger immune response than transplantation of cell suspension because of the remaining donor blood vessels and antigen-presenting cells (Non Patent Literature 5). On the other hand, if the immune response is suppressed by isogeneic transplantation or an immunosuppressive agent, this problem is eliminated, and transplantation of ventral midbrain (VM) tissue shows dopamine-producing nerves having a higher survival rate and behavioral recovery than transplantation of cell suspension (Non Patent Literature 6). In addition, mechanical and enzymatic dissociation processes to obtain a cell suspension can alter cell properties to cause cell injury. Therefore, in clinical applications, it is desirable that cells to be transplanted should be administered as a sphere rather than a cell suspension. However, spheres are more difficult to cryopreserve than single cells.

When cryopreserved PSC-derived dopamine-producing nerves as a single cell suspension is transplanted into rat corpus striatum, the survival ratio of TH⁺ cells is reduced to about 60% compared to unfrozen cells (Non Patent Literature 7). On the other hand, in most researches with human or rat ventral midbrain (VM) tissue cryopreserved, the dopamine-producing nerves *in vivo* have a survival rate reduced to less than 20% compared to unfrozen tissue (Non Patent Literatures 8 to 10). Therefore, there has been a need to develop a freezing method that can maintain the survival of a PSC-derived dopamine-producing neuron sphere.

In general, there are two cell cryopreservation methods (Non Patent Literatures 11 to 13). The slow cooling method of these methods is a method in which cells are frozen together with a low concentration of cryoprotectant (CPA) (such as 10% dimethyl sulfoxide (DMSO)) at about 1 °C/min (Patent Literature 5, Non Patent Literatures 14 and 15). On the other hand, the vitrification method is an ultra-fast cooling method in which cells are transferred into liquid nitrogen immediately after a high concentration of cryoprotectant is added (Patent Literature 6, Non Patent Literature 16). Since the vitrification method requires strict time control, its application to clinical cell production has been technically difficult (Non Patent Literature 17).

In the slow cooling method, on the other hand, ice formation begins first in the extracellular space, leading to the concentration of extracellular fluid. As a result, water is withdrawn from the cells by an osmotic gradient across the cell membrane. This dehydration of the cells avoids intracellular ice formation. However, if cells are excessively dehydrated, they are injured by the concentrated intracellular fluid and by the CPA in the cryopreservation solution. Because of the need for precise control of ice formation and cell dehydration, no clinical cryopreservation method for a sphere has been established.

### Citation List

### Patent Literature

[Patent Literature 1]
   JP2017-104061A
[Patent Literature 2]
   WO2017/159862
[Patent Literature 3]
   JP2015-521469A
[Patent Literature 4]
   JP2008-501320A
[Patent Literature 5]
   JP2011-103885A
[Patent Literature 6]
   JP2013-110988A

### Non Patent Literature

[Non Patent Literature 1]
   Piccini et al; Nature Neuroscience, 2(12), 1137-1140, 1999
[Non Patent Literature 2]
   Doi et al., Stem Cell Reports, 2(3), 337-350, 2014
[Non Patent Literature 3]
   Sundberg et al., Stem Cells 31, 1548-1562, 2013
[Non Patent Literature 4]
   Nolbrant et al., Nature Protocols, 12(9), 1962-1979, .2017
[Non Patent Literature 5]
   Redmond et al., Neurobiology of Disease, 29(1), 103-116, 2008
[Non Patent Literature 6]
   Fricker et al., PLoS ONE, 7(10), e47169, 2012
[Non Patent Literature 7]
   Nolbrant et al., Nature Protocols, 12(9), 1962-1979, 2017
[Non Patent Literature 8]
   Frodl et al., Brain Research, 647(2), 286-298, 1994
[Non Patent Literature 9]
   Sautter et al., Journal of Neuroscience Methods, 64(2), 173-179, 1996
[Non Patent Literature 10]
   Sautter et al., Experimental Neurology, 164(1), 121-129, 2000
[Non Patent Literature 11]
   Chong et al., Stem Cells, 27(1), 29-39, 2009
[Non Patent Literature 12]
   Smith et al., Fertility and Sterility, 94(6), 2088-2095, 2010
[Non Patent Literature 13]
   Jang et al., Integrative Medicine Research, 6(1), 12-18, 2017
[Non Patent Literature 14]
   Schwartz et al., Journal of Neuroscience Research, 74(6), 838-851, 2003
[Non Patent Literature 15]
   Woods et al., Cryobiology, 59(2), 150-157, 2009
[Non Patent Literature 16]
   Fahy and Wowk., Methods in Molecular Biology (Methods and Protocols), vol 1257. Springer, New York, NY, 2015
[Non Patent Literature 17]
   Nagano et al., Biomedical Research, 28(3), 153-160, 2007

### SUMMARY OF INVENTION

### TECHNICAL PROBLEM

An object of the present application is to provide a method for freezing cell aggregates including neural cells.

### SOLUTION TO PROBLEM

As a result of intensive studies, the present inventors have found a method for freezing a cell aggregate including neural cells and have completed the present invention. More specifically, the present invention relates to the following:
[1] A method for freezing a cell aggregate including neural cells and having a three-dimensional structure, which comprises following steps (1) and (2):
   (1) contacting the cell aggregate including neural cells and having a three-dimensional structure with a preservation solution at 0°C to 30°C prior to freezing to prepare a preservation solution-soaked cell aggregate; and
   (2) cooling the preservation solution-soaked cell aggregate obtained in step (1) from a temperature at least about 5°C higher than the freezing point of the preservation solution to a temperature about 5°C lower than the freezing point at an average cooling speed of 2 to 7 °C/min to freeze the cell aggregate.
[2] The method according to [1], wherein the average cooling speed in step (2) is 3 to 7 °C/min.
[3] The method according to [1] or [2], wherein the cell aggregate is contacted with the preservation solution for 15 minutes to 90 minutes, preferably 15 minutes to 60 minutes in step (1).
[4] The method according to any one of [1] to [3], wherein the preservation solution has the freezing point of from -1°C to -10°C.
[5] The method according to any one of [1] to [4], wherein the preservation solution is an aqueous solution comprising 7% to 12% of dimethyl sulfoxide and/or propylene glycol, and step (2) is a step of cooling from 0 ± 5°C to -30 ± 5°C at an average cooling speed of 2 to 5 °C/min.
[6] The method according to any one of [1] to [5], wherein the average cooling speed in step (2) is 3 to 5 °C/min.
[7] The method according to any one of [1] to [6], wherein the method further comprises a following step (3):
   (3) cooling the frozen cell aggregate obtained in step (2) to -50°C or lower.
[8] The method according to any one of [1] to [7], wherein the cell aggregate including neural cells is a cell aggregate including neural cells derived from pluripotent stem cells.
[9] The method according to any one of [1] to [8], wherein the cell aggregate including neural cells comprises cells that are positive for at least one of FOXA2, TH, and NURR1.
[10] The method according to [9], wherein the cell aggregate including neural cells comprises cells positive for FOXA2 and LMX1A.
[11] The method according to [9], wherein the cell aggregate including neural cells comprises cells positive for FOXA2, TH, and NURR1.
[12] The method according to any one of [1] to [8], wherein the cell aggregate including neural cells comprises cells positive for FOXA2 and LMX1A in an amount of 40% or more of the total cells and cells positive for TH and NURR1 in an amount of 40% or less of the total cells.
[13] The method according to any one of [1] to [12], wherein the cell aggregate including neural cells comprises a dopamine-producing neuron progenitor cell and/or a dopamine-producing neuron.
[14] The method according to any one of [1] to [13], wherein the cell aggregate includes 500 to 150000 cells.
[15] The method according to any one of [1] to [14], wherein number of cells contained in the preservation solution is 80000 to 5000000 cells/mL, and the cell aggregate has an equivalent spherical diameter of 150 to 1000 µm.
[16] The method according to any one of [1] to [15], wherein the cell aggregate and the preservation solution have volume of 0.25 mL to 2 mL.
[17] The method according to any one of [1] to [16], wherein the cell aggregate and the preservation solution are packed in a 0.5 mL to 15 mL container.
[18] A method for preserving a cell aggregate including neural cells and having a three-dimensional structure for a long-term, wherein the method comprises holding the frozen cell aggregate obtained by the method according to any one of [1] to [17] at or below -80°C.
[19] The method according to any one of [1] to [18], wherein the obtained frozen cell aggregate can be used without recovery culture after thawing.
[20] A composition for transplantation, wherein the composition comprises, as an active ingredient, the cell aggregate frozen or preserved for a long-term by the method according to any one of [1] to [19].
[21] A frozen composition for transplantation, wherein the composition comprises:
   a cell aggregate including 60% or more of dopamine-producing neuron progenitor cells and dopamine-producing neurons derived from a pluripotent stem cell, having an equivalent spherical diameter of 150 µm to 1000 µm and including 500 to 150000 cells; and
   a cryopreservation solution comprising 7% to 12% of dimethyl sulfoxide or propylene glycol and having the freezing point of -1°C to -10°C, and
   wherein the frozen cell aggregate has following properties:
      (1) about 60% or more of the total cells are viable after thawing;
      (2) the cells after thawing have a neurite extension activity of 50% or more of that before freezing; and
      (3) the rates of the FOXA2-positive cells, LMX1A-positive cells, NURR1-positive cells, and TH-positive cells in the cells viable after thawing are changed within ± 10% from those in the cells before freezing.
[22] The composition for transplantation according to [20] or [21], wherein number of cells in the composition is 80000 to 5000000 cells/mL, and the composition comprises cells positive for FOXA2 and LMX1A in an amount of 40% or more of the total cells and cells positive for TH and NURR1 in an amount of 40% or less of the total cells.
[23] The composition for transplantation according to any one of [20] to [22], wherein the cell aggregate has an equivalent spherical diameter of 150 to 1000 µm.
[24] The composition for transplantation according to any one of [20] to [23], wherein the composition can be used without recovery culture after thawing.
[25] The composition for transplantation according to any one of [20] to [24], wherein the composition comprises 8 to 192 cell aggregates/mL, the cell aggregate has an equivalent spherical diameter of 150 µm to 1000 µm, and number of cells per container is 80000 to 2400000.
[26] The composition for transplantation according to any one of [20] to [25], wherein the cell aggregate and the preservation solution have volume of 0.25 mL to 2 mL.
[27] The composition for transplantation according to any one of [20] to [26], wherein the composition is packed in a 0.5 mL to 15 mL container.
[28] A method for producing a composition for transplantation containing a dopamine-producing neuron progenitor cell as an active ingredient, which comprises:
   freezing a cell aggregate by the method according to any one of [1] to [17], wherein number of cells in the composition is 80000 to 5000000 cells/mL, the cell aggregate comprises cells positive for FOXA2 and LMX1A in an amount of 40% or more of the total cells and cells positive for TH and NURR1 in an amount of 40% or less of the total cells, and the cell aggregate has an equivalent spherical diameter of 150 µm to 1000 µm.
[29] The method for producing the composition for transplantation according to [28] containing an aggregate of neural cells as an active ingredient, wherein the composition comprises a population of cell aggregates which have an equivalent spherical diameter of 150 to 1000 µm, number of cells per container is 80000 to 2400000, the cell aggregate and the preservation solution have volume of 0.25 mL to 2 mL, and the composition is packed in a 0.5 mL to 15 mL container.
[30] A method for treating a disease requiring regeneration of a dopamine-producing nerve, which comprises following steps of:
   (1) thawing the composition for transplantation according to any one of [20] to [27] at 30°C to 40°C, preferably at 37°C ± 3°C; and
   (2) transplanting the composition for transplantation obtained in (1) into a corpus striatum region of a patient.
[31] The method according to [30], wherein the cryopreservation solution is replaced with a dosing vehicle without culture after thawing to perform step (2).

### EFFECTS OF THE INVENTION

The present application provides a method for cryopreserving a cell aggregate including neural cells. The neural cells cryopreserved by the method of the present application exhibit high cell viability and maintain functional properties.

### BRIEF DESCRIPTION OF DRAWINGS

[Figure 1] A scheme showing a protocol for inducing differentiation from iPSCs into dopamine-producing neuron progenitor cells and timings for evaluations. Abbreviations in the figure indicate components added to medium. LDN: LDN193189, A: A83-01, Y: Y-27632, Pur: Purmorphamine, CHIR: CHIR99021, AA: ascorbic acid.
[Figure 2A] The effect of cryopreservation solutions on iPSC-derived dopamine-producing neuron progenitor cells. Recovery of viable cells after thawing from unsorted cells that were immersed in the cryopreservation solutions shown in Table 1 for 15 minutes and then cryopreserved at 0.5 °C/min (n = 4).
[Figure 2B] The effect of cryopreservation solutions on iPSC-derived dopamine-producing neuron progenitor cells. Neurite extension of spheres derived from the unsorted cells that were immersed in the cryopreservation solutions shown in Table 1 for 15 minutes and then cryopreserved at 0.5 °C/min (n = 4) .
[Figure 2C] The effect of a cryopreservation solutions on iPSC-derived dopamine-producing neuron progenitor cells. Shown is immunostained images obtained by staining, with an early neuronal marker (PSA-NCAM), neurites of spheres derived from unsorted cells that were immersed in the cryopreservation solutions shown in Table 1 for 15 minutes and then cryopreserved at 0.5 °C/min. The scale bar indicates 1 mm.
[Figure 3] Time-temperature curves for a sample (straight line), a freezing chamber (dashed line), and a program (dotted line). Bambanker hRM was used as the sample. The lower graph shows an enlargement of the temperature change caused by latent heat release in the upper graph.
[Figure 4A] The effect of cryopreservation programs on iPSC-derived dopamine-producing neuron progenitor cells. Recovery of viable cells after thawing from unsorted cells that were immersed in Bambanker hRM for 15 minutes and then cryopreserved according to different freezing programs.
[Figure 4B] The effect of a cryopreservation programs on iPSC-derived dopamine-producing neuron progenitor cells. Neurite extension of spheres derived from the unsorted cells that were immersed in Bambanker hRM for 15 minutes and then cryopreserved according to different freezing programs.
[Figure 5A] The effect of freezing iPSC-derived dopamine-producing neuron progenitor cells after prolonged exposure to the cryopreservation solutions. Recovery of viable cells after thawing in unsorted cells that were exposed to Bambanker hRM for 60 minutes and then cryopreserved in the different freezing programs.
[Figure 5B] The effect of freezing iPSC-derived dopamine-producing neuron progenitor cells after prolonged exposure to the cryopreservation solutions. Neurite extension of spheres derived from the unsorted cells that were exposed to Bambanker hRM for 60 minutes and then cryopreserved in the different freezing programs.
[Figure 6A] *In vitro* characteristics of the cryopreserved sphere. Recovery of viable cells after thawing.
[Figure 6B] *In vitro* characteristics of the cryopreserved sphere. Neurite extension of the sphere.
[Figure 6C] *In vitro* characteristics of the cryopreserved sphere. Immunostaining of the sphere at day 35 and day 7 after thawing. The left panels show immunostaining images for FOXA2/DAPI, the center panels show immunostaining images for NURR1/TH, and the right panels show immunostaining images for SOX1/KI67/PAX6/DAPI. The scale bar indicates 100 µm.
[Figure 6D] *In vitro* characteristics of the cryopreserved sphere. Percentages of FOXA2⁺ cells, NURR1⁺ cells, and TH⁺ cells to total cells at day 35 and day 7 after thawing.
[Figure 6E] *In vitro* characteristics of the cryopreserved sphere. Percentages of SOX1⁺ cells, PAX6⁺ cells, and KI67⁺ cells to total cells at day 35 and day 7 after thawing.
[Figure 6F] *In vitro* characteristics of the cryopreserved sphere. The gene expression of the spheres relative to GAPDH measured by quantitative RT-PCR. The expression level of undifferentiated cells (day 0) was set to 1.
[Figure 6G] *In vitro* characteristics of the cryopreserved sphere. The gene expression of the spheres relative to GAPDH measured by quantitative RT-PCR. The expression level of undifferentiated cells (day 0) was set to 1.
[Figure 6H] *In vitro* characteristics of the cryopreserved sphere. A principal component analysis of the microarray data. Temporal changes in gene expressions of unfrozen cells (circle) and frozen cells (triangle) are shown.
[Figure 6I] *In vitro* characteristics of the cryopreserved sphere. Scatter plots of the microarray data from unfrozen cells and frozen cells in the same lot at day 35 (X-axis) or day 7 after thawing (Y-axis) are shown. A solid black circle indicates a gene showing a signal intensity of 50 or more in either one of the samples, and a white circle indicates a gene showing a signal intensity of 50 or less in both samples.
[Figure 6J] *In vitro* characteristics of the cryopreserved sphere. Scatter plots of microarray data from unfrozen cells in different lots at day 35 are shown. A solid black circle indicates a gene showing a signal intensity of 50 or more in either one of the samples, and a white circle indicates a gene showing a signal intensity of 50 or less in both samples.
[Figure 6K] *In vitro* characteristics of the cryopreserved sphere. Immunostaining images for TUBB3, TH, and DAPI in iPSC-derived dopamine-producing neurons at day 28 + 21 after thawing. The scale bar indicates 50 µm.
[Figure 6L] *In vitro* characteristics of the cryopreserved sphere. A representative induced action potential of iPSC-derived dopamine-producing neurons at day 28 + 21 after thawing.
[Figure 6M] *In vitro* characteristics of the cryopreserved sphere. Results of dopamine release induced by high potassium stimulation at day 56 or day 28 + 28 after thawing.
[Figure 7A] Temporal change in the expression of a dopamine-producing neuron progenitor cell marker after thawing. Percentage of FOXA2⁺ cells to total cells at days 28, 29, 31, and 35 and days 0, 1, 3, and 7 after thawing.
[Figure 7B] Temporal change in the expression of a dopamine-producing neuron progenitor cell marker after thawing. Percentage of NURR1⁺ cells to total cells at days 28, 29, 31, and 35 and days 0, 1, 3, and 7 after thawing.
[Figure 7C] Temporal change in the expression of a dopamine-producing neuron progenitor cell marker after thawing. The gene expression for TH of the sphere relative to GAPDH measured by quantitative RT-PCR. The expression level of undifferentiated cells (day 0) was set to 1.
[Figure 8A] The graft survival rate and function of a cryopreserved sphere. Methamphetamine-induced rotational movements of rats having grafts. Data are presented as the mean ± SEM (n = 6 to 8). Two-way ANOVA with Tukey's multiple comparison test was performed, and significance levels are indicated by **p <0.01, ****p <0.001 relative to a solvent-administered group.
[Figure 8B] The graft survival rate and function of a cryopreserved sphere. Immunostaining images for HNA of representative grafts derived from unfrozen cells (upper panel) and frozen cells (lower panel).
[Figure 8C] The graft survival rate and function of a cryopreserved sphere. Number of survived HNA⁺ cells in grafts.
[Figure 8D] The graft survival rate and function of a cryopreserved sphere. A DAB-staining image for TH of a representative graft derived from unfrozen cells.
[Figure 8E] The graft survival rate and function of a cryopreserved sphere. A magnified image within the frame of Figure 8D. The right panel is a magnified image within the frame of the left panel. The scale bar indicates 200 µm.
[Figure 8F] The graft survival rate and function of a cryopreserved sphere. A DAB-staining image for TH of a representative graft derived from frozen cells.
[Figure 8G] The graft survival rate and function of a cryopreserved sphere. A magnified image within the frame of Figure 8F. The right panel is a magnified image within the frame of the left panel. The scale bar indicates 200 µm.
[Figure 8H] The graft survival rate and function of a cryopreserved sphere. Number of survived TH⁺ cells in grafts.
[Figure 8I] The graft survival rate and function of a cryopreserved sphere. Immunostaining images for FOXA2, TH, and HNA (upper), and KI67 and HNA (lower) of grafts derived from frozen cells. The scale bar indicates 50 µm.
[Figure 8J] The graft survival rate and function of a cryopreserved sphere. Percentages of FOXA2⁺ cells relative to HNA⁺ cells.
[Figure 8K] The graft survival rate and function of a cryopreserved sphere. Percentages of KI67⁺ cells relative to HNA⁺ cells.
[Figure 9] The survival rate and neurite extension activity of cryopreserved spheres thawed under different conditions.
[Figure 10A] The expression of markers in a sphere derived from cells not sorted with anti-CORIN antibodies. Immunostaining images for LMX1A, FOXA2, and DAPI (upper), NURR1, TH, and DAPI (center), and SOX1, KI67, PAX6, and DAPI (lower) of the sphere. The scale bar indicates 100 µm.
[Figure 10B] The expression of markers in a sphere derived from cells not sorted with anti-CORIN antibodies. Percentages of FOXA2⁺/LMXlA⁺ cells, NURR1⁺ cells, and TH⁺ cells relative to total cells.
[Figure 10C] The expression of markers in a sphere derived from cells not sorted with anti-CORIN antibodies. Percentages of SOX1+ cells, PAX6⁺ cells, and KI67⁺ cells relative to total cells.
[Figure 11] The expression of markers in a sphere immediately before freezing (day 28) in the same lot as the sphere shown in Figure 6C. Immunostaining images for LMX1A, FOXA2, and DAPI (upper), NURR1, FOXA2, TH, and DAPI (center), and SOX1, KI67, PAX6, and DAPI (upper) of spheres. The scale bar indicates 100 µm.

### DESCRIPTION OF EMBODIMENTS

In the present specification and claims, when a numerical value is accompanied by the term "about," it is intended to include a range of ± 10% of the value. For example, "about 20" shall include "18 to 22". A numerical range includes all numerical values between the two endpoints and the numerical values at both endpoints. The term "about" for a range applies to both endpoints of the range. Thus, for example, "about 20 to 30" shall include "18 to 33".

### [Neural cells]

The present application provides a method for freezing a cell aggregate including neural cells and having a three-dimensional structure.

The neural cells include neuronal cells or neurons, progenitor cells of the neurons, i.e., neural progenitor cells or neural precursor cells.

The neural cells may be neural cells derived from any site such as neural cells of the central nervous system, or neural cells of the peripheral nervous system such as somatic neural cells of motor nerves or sensory systems or neural cells of autonomic nerves; examples of the neural cells include nerves (neurons), neural crest-derived cells, glia cells such as oligodendrocytes or astrocytes, and stem cells or progenitor cells thereof. Examples of the neural cell include a cell expressing a neural cell marker. Examples of the neural cell marker include, but are not limited to, NCAM, βIII-Tubulin (TUJ1), tyrosine hydroxylase (TH), serotonin, nestin, MAP2, MAP2AB, NEUN, GABA, glutamate, CHAT, SOX1, BF1, EMX1, VGLUT1, PAX, NKX, GSH, Telencephalin, GLUR1, CAMKII, CTIP2, TBR1, Reelin, TBR1, BRN2, OTX2, LMX1A, LMX1B, EN1, NURR1, PITX3, DAT, GIRK2, and TH. The neural cell can be identified by the expression of one or more neural cell markers. In the present specification, examples of the neural cell include a cell expressing one or more, two or more, or three or more of the above neural cell markers.

Neural cells of the central nervous system can be classified according to differences in sites where the neural cells nervous systems are present. That is, examples of the neural cells of the central nervous system include neurons derived from prosencephalon, telencephalon, diencephalon, cerebral, hypothalamus, mesencephalon, metencephalon, mesencephalon-metencephalon boundary region, cerebellum, retina, pituitary gland, or spinal cord, and progenitor cells thereof.

The neurons derived from prosencephalon are neurons that are present in prosencephalon tissue (i.e., telencephalon, cerebral, hippocampus or choroid plexus, diencephalon, hypothalamus, etc.). The neurons of prosencephalon can be identified by the expression of a prosencephalon neuron marker. Examples of the prosencephalon neuron marker include OTX1 (prosencephalon), BF1 (also referred to as FOXG1) or SIX3 (also serving as a marker for telencephalon or cerebral). In the present specification, examples of the neural cell include a cell expressing one or more, two or more, or three or more of the above prosencephalon neuron markers or the markers for telencephalon or cerebral.

Examples of the neuron derived from cerebral include dorsal cells (e.g., cerebral cortex cells, Cajal-Retzius cells, hippocampus neurons) and ventral cells (e.g., basal ganglia). Examples of the ventral cerebral neuron marker include basal ganglia neuron markers (e.g., GSH2, MASH1, NKX2.1, NOZ1). Examples of the dorsal cerebral neuron marker include cerebral cortex neuron markers (e.g., PAX6, EMX1, TBR1). In the present specification, examples of the neural cell include a cell expressing one or more, two or more, or three or more of the above cerebral neuron markers, the basal ganglia neuron markers or the cerebral cortex neuron markers.

Examples of the neural cell derived from mesencephalon include neural progenitor cells derived from ventral mesencephalon, dopamine-producing neurons (also referred to as dopaminergic neurons) and dopamine-producing neuron progenitor cells (also referred to as dopaminergic neuron progenitor cells or dopaminergic progenitors). Examples of a mesencephalon-derived neural cell marker include FOXA2, EN2, and TUJ1. Examples of a FOXA2-positive and TUJ1-positive neural cell include dopamine-producing neuron progenitor cells and dopamine-producing neurons. The dopamine-producing neurons can be identified by being FOXA2-positive, NURR1-positive, and TH-positive.

The dopamine-producing neuron progenitor cells can be identified by being FOXA2-positive and LMX1A-positive. The dopamine-producing neuron progenitor cells more preferably contain cells that are positive for one or more of OTX2, LMX1A, LMX1B, CORIN, SHH, AADC, βIII-Tubulin, EN1, NURR1, PITX3, DAT, GIRK2, and TH. In the present specification, a cell aggregate including dopamine-producing neuron progenitor cells, unless otherwise specified, may comprise dopamine-producing neurons or dopaminergic neurons.

In the present specification, examples of the neural cell include a cell expressing one or more, two or more, or three or more of the mesencephalon-derived neural cell markers, the dopamine-producing neuron progenitor cell markers or the dopamine-producing neuron markers.

In the present specification, examples of the dopamine-producing neuron progenitor cells include cells expressing FOXA2 and/or LMX1A (FOXA2-positive and/or LMX1A-positive), preferably cells expressing one or more, two or more, or three or more selected from the group consisting of OTX2, LMX1B, CORIN, SHH, AADC, and βIII-Tubulin in addition to FOXA2 and LMXA1.

In the present specification, examples of the dopamine-producing neurons (dopaminergic neurons) include cells expressing TH and/or NURR1 (TH-positive and/or NURR1-positive), preferably cells expressing one or more, two or more, or three or more selected from the group consisting of FOXA2, AADC, DAT, and GIRK2 in addition to TH and NURR1.

Examples of the neurons derived from the mesencephalon-metencephalon boundary region include neurons that are present in the cerebellum, cerebellar plate tissue, ventricular zone, rhombic lip, etc. Examples of a mesencephalon-metencephalon boundary region marker include EN2 (mesencephalon), GBX2 (metencephalon), N-Cadherin (neural progenitor cells in the mesencephalon-metencephalon boundary region). Examples of a cerebellar neural progenitor cell marker include KIRREL2, PTF1A, and SOX2, which are GABAergic progenitor cell markers, and ATOH1 and BARHL1, which are cerebellar granule cell progenitor cell markers. In the present specification, examples of the neural cell include a cell expressing one or more, two or more, or three or more of the above mesencephalon-metencephalon boundary region markers, the cerebellar neural progenitor cell markers, the GABAergic progenitor cell markers, or the cerebellar granule cell progenitor cell markers.

Examples of a neural cell derived from a retina include photoreceptor cells, photoreceptor progenitor cells, retinal pigment epithelium cells, and cornea cells.

The neural cells can also be classified according to differences in neurotransmitters produced (secreted); examples thereof include dopamine-producing neurons, dopamine-producing neuron progenitor cells, GABAergic neurons, GABAergic progenitor cells, cholinergic neurons, cholinergic progenitor cells, serotonergic neurons, serotonergic progenitor cells, glutamatergic neurons, glutamatergic progenitor cells, noradrenergic neurons, noradrenergic progenitor cells, adrenergic neurons, and adrenergic progenitor cells.

Examples of the neural cells of motor nerves or sensory systems include cholinergic neurons and progenitor cells thereof.

Examples of the neural cells of autonomic nerves include cholinergic neurons, adrenergic neurons, and progenitor cells thereof.

Preferred examples of the neural cells in the present specification include dopamine-producing neurons (dopaminergic neurons), and dopamine-producing neuron progenitor cells (dopaminergic neuron progenitor cells).

Neural cells derived from a living body are cells isolated from a mammal such as humans, and examples of the cells isolated from human brain tissue include cells contained in the fetal mesencephalon tissue as described in Nature Neuroscience, 2, 1137 (1999) or N. Engl. J. Med.; 344: 710-9 (2001).

The neural cells may also be cells obtained by inducing differentiation from pluripotent stem cells such as embryonic stem cells (ES cells) and iPS cells. Examples of a method for inducing differentiation from pluripotent stem cells into neural cells include the methods described in Non Patent Literatures 3 and 4 and WO2015/034012 (dopamine-producing neuron progenitor cells), WO2009/148170 (cerebral neural cells and the like), WO2013/065763, WO2016/013669 or WO2017/126551 (hypophysial or subthalamic neural cells), WO2016/039317 (cerebellar neural cells), WO2015/076388 (telencephalic neural cells), Numasawa-Kuroiwa, Y et al., Stem Cell Reports, 2: 648-661 (2014) (neural progenitor cells), Qiu, L et al., Stem Cells Transl Med. 6 (9): 1803-1814 (2017) (dopamine-producing neuron progenitor cells).

The neural cells may be cells obtained by inducing differentiation from multipotent stem cells such as mesenchymal stem cells (MSCs). Examples of a method for inducing differentiation from mesenchymal stem cells into neural cells include the method described in J Chem Neuroanat. 96: 126-133 (2019).

### [Pluripotent stem cell]

A pluripotent stem cell refers to a stem cell that has both pluripotency that enables differentiation into almost all cells existing in a living body and proliferative capacity. Pluripotent stem cells can be derived from fertilized ova, cloned embryos, germ stem cells, interstitial stem cells, or somatic cells. Examples of the pluripotent stem cell include, but are not particularly limited to, embryonic stem (ES) cells, nuclear transfer embryonic stem (ntES) cells derived from cloned embryos, spermatogonial stem cells (GS cells), embryonic germ cells (EG cells), induced pluripotent stem (iPS) cells, pluripotent stem cells derived from cultured fibroblasts and bone marrow stem cells (Muse cells). The pluripotent stem cells may be ES cells, ntES cells, or iPS cells. The pluripotent stem cells may be iPS cells in view of ethical considerations. Note that the embryonic stem cells are established from embryos within 14 days of fertilization.

Embryonic stem cells were first established in 1981 and have been applied to the production of knockout mice since 1989. Human embryonic stem cells were established in 1998 and are being used in regenerative medicine. Embryonic stem cells can be produced by culturing an inner cell mass on feeder cells or in medium containing leukemia inhibitory factor (LIF). Methods for producing embryonic stem cells are described, for example, in WO96/22362, WO02/101057, US5,843,780, US6,200,806, US6,280,718, etc. Embryonic stem cells can be obtained from a given institution or purchased commercially. For example, human embryonic stem cells KhES-1, KhES-2, and KhES-3 are available from Institute for Frontier Life and Medical Sciences, Kyoto University. Human embryonic stem cell line Rx::GFP (derived from the KhES-1 line) is available from Institute of Physical and Chemical Research. Mouse embryonic stem cell lines, EB5 and D3, are available from Institute of Physical and Chemical Research and ATCC, respectively.

A nuclear transfer embryonic stem cell (ntES cell), which is one type of embryonic stem cell, can be established from a cloned embryo created by transferring the nucleus of a somatic cell into an ovum from which the nucleus has been removed.

EG cells can be produced by culturing primordial germ cells in medium containing mSCF, LIF, and bFGF (Cell, 70: 841-847, 1992).

The term "induced pluripotent stem cell" as used herein refers to a cell obtained by reprogramming a somatic cell to have pluripotency induced using a known method. Specific examples thereof include cells obtained by reprogramming differentiated somatic cells such as fibroblasts or peripheral blood mononuclear cells by expression of any combination of a plurality of genes selected from a reprogramming gene group including OCT3/4, SOX2, KLF4, MYC (c-MYC, N-MYC, L-MYC), GLIS1, NANOG, SALL4, LIN28, ESRRB to induce pluripotency. Preferred examples of the combination of reprogramming factors include (1) OCT3/4, SOX2, KLF4, and MYC (c-MYC or L-MYC), (2) OCT3/4, SOX2, KLF4, LIN28, and L-MYC (Stem Cells, 2013; 31: 458 to 466), and (3) OCT3/4, SOX2, NANOG, and LIN28 (Science 2007; 318: 1917 to 1920).

Induced pluripotent stem cells were established with mouse cells by Yamanaka et al. in 2006 (Cell, 2006, 126 (4), pp. 663 to 676). Induced pluripotent stem cells were also established with human fibroblasts in 2007 and have pluripotency and replication competence similar to embryonic stem cells (Cell, 2007, 131 (5), pp. 861 to 872; Science, 2007, 318 (5858), pp. 1917 to 1920; Nat. Biotechnol., 2008, 26 (1), pp. 101 to 106).

Induced pluripotent stem cells can also be produced by a method of deriving induced pluripotent stem cells from somatic cells through the addition of a compound or the like, in addition to a method of producing induced pluripotent stem cells by reprogramming directly through gene expression (Science, 2013, 341, pp. 651 to 654).

It is also possible to obtain an established induced pluripotent stem cell line; for example, human induced pluripotent stem cell lines such as 201B7 cells, 201B7-Ff cells, 253G1 cells, 253G4 cells, 1201C1 cells, 1205D1 cells, 1210B2 cells, 1231A3 cells, etc. established at Kyoto University are available from Kyoto University. For example, Ff-I01 cells, Ff-I01s04 cells, QHJ-I01 and Ff-I14 cells established at Kyoto University are available from Kyoto University as the established induced pluripotent stem cell line.

Examples of somatic cells used in the production of induced pluripotent stem cells include, but are not particularly limited, tissue-derived fibroblasts, erythroid cells (e.g., peripheral blood mononuclear cells (PBMCs), T cells), hepatocytes, pancreatic cells, enterocytes, and smooth muscle myocytes.

For reprogramming through expression of several genes in the production of induced pluripotent stem cells, the means for expressing the genes is not particularly limited. Examples of the above-mentioned means include infection with a viral vector (e.g., retroviral vectors, lentiviral vectors, Sendai viral vectors, adenoviral vectors, or adeno-associated virus vectors), gene transfer (e.g., calcium phosphate transfection, lipofection, RetroNectin method, or electroporation) using a plasmid vector (e.g., plasmid vectors or episomal vectors), gene transfer (e.g., calcium phosphate transfection, lipofection, or electroporation) using an RNA vector, and direct injection (e.g., needle-based method, lipofection, or electroporation) of a protein.

Induced pluripotent stem cells can be produced in the presence of feeder cells or in the absence of feeder cells (feeder-free). In the production of induced pluripotent stem cells in the presence of feeder cells, the induced pluripotent stem cells can be produced in the presence of an undifferentiated-state maintenance factor by a known method. Medium used to produce induced pluripotent stem cells in the absence of feeder cells is not particularly limited and can be any known maintenance medium for embryonic stem cells and/or induced pluripotent stem cells, or any medium for establishing induced pluripotent stem cells in a feeder-free manner. Examples of the medium for establishing induced pluripotent stem cells in a feeder-free manner include feeder-free media such as Essential 8 medium (E8 medium), Essential 6 medium, TeSR medium, mTeSR medium, mTeSR-E8 medium, Stabilized Essential 8 medium, and StemFit medium. In the production of induced pluripotent stem cells, for example, four factors, OCT3/4, SOX2, KLF4, and MYC (L-MYC or C-MYC) can be transferred into somatic cells in a feeder-free manner using Sendai viral vectors . to prepare the induced pluripotent stem cells.

Pluripotent stem cells used in the present invention are mammalian pluripotent stem cells, preferably rodent (e.g., mouse or rat) or primate (e.g., human or monkey) pluripotent stem cells, more preferably human or mouse pluripotent stem cells, even more preferably human induced pluripotent stem cells (iPS cells) or human embryonic stem cells (ES cells).

### [Cell aggregate]

The cell aggregate "having a three-dimensional structure" herein refers to a cell aggregate (cell aggregate or sphere) which is a three-dimensional cell population formed by cultured cells adhering to each other through, for example, a suspension culture or a 3D culture. A cell aggregate of neural cells is also referred to as a neurosphere. The shape of the cell aggregate is not particularly limited and may be spherical or non-spherical. The cell aggregate in the present specification is preferably a cell aggregate having a three-dimensional shape similar to a sphere. The three-dimensional shape similar to a sphere is a shape having a three-dimensional structure and shows, for example, a circular shape or an elliptical shape when projected onto a two-dimensional plane.

The cell aggregate including neural cells and having a three-dimensional structure has no particular restrictions on its size but usually has an equivalent spherical diameter of 150 µm to 1000 µm, and for example, 200 µm to 800 µm or 300 µm to 500 µm in one embodiment. The cell aggregate including neural cells and having a three dimensional structure usually includes 500 to 150000 cells, and in one embodiment, for example, 1000 to 100000 cells, 1000 to 70000 cells, or 3000 to 30000 cells.

The cell aggregate including neural cells may comprise other cells together with the neural cells. Examples of such a cell aggregate include a cell aggregate including 60% or more, 70% or more, 80% or more, and more preferably 90% or more of neural cells.

In one embodiment, the cell aggregate including neural cells may comprise 60% or more, 70% or more, or 80% or more of dopamine-producing neuron progenitor cells and/or dopamine-producing neurons. That is, the cell aggregate including neural cells may comprise neural cells expressing one or more markers selected from FOXA2, LMX1A, LMX1B, NURR1, and TH in an amount of 60% or more, 70% or more, or 80% or more.

In one embodiment, the cell aggregate including neural cells comprises 40% or more, 60% or more, 70% or more, 80% or more, 85% or more, or 90% or more of dopamine-producing neuron progenitor cells.

In one embodiment, the cell aggregate including neural cells comprises cells expressing one or more, two or more, or three or more markers for dopamine-producing neuron progenitor cells in an amount of 40% or more, 60% or more, 70% or more, 80% or more, 85% or more, or 90% or more.

In one embodiment, the cell aggregate including neural cells comprises cells positive for FOXA2 and LMX1A in an amount of 40% or more, 60% or more, 70% or more, 80% or more, 85% or more, or 90% or more. In one embodiment, the cell aggregate further comprises cells positive for TH and NURR1 in an amount of 40% or less.

In one embodiment, the cell aggregate including neural cells may comprise cells positive for FOXA2, TH, and NURR1 in an amount of 0% or more, 10% or more, or 20% or more.

In one embodiment, the cell aggregate including dopamine-producing neuron progenitor cells may comprise NURR1-positive cells in an amount of 60% or less, 50% or less, 40% or less, 5 to 50%, 5 to 40%, or 5 to 20%.

In one embodiment, the cell aggregate including dopamine-producing neuron progenitor cells and/or dopamine-producing neurons may comprise TH-positive cells in an amount of 30% or less, 20% or less, 1 to 30%, 5 to 30%, 1 to 20%, 5 to 20%, or 5 to 15%.

In one embodiment, the cell aggregate including dopamine-producing neuron progenitor cells and/or dopamine-producing neurons may comprise KI67-positive cells in an amount of 30% or less, 1 to 25%, 1 to 20%, or 5 to 20%.

In one embodiment, the cell aggregate including dopamine-producing neuron progenitor cells and/or dopamine-producing neurons may comprise SOX1-positive cells in an amount of 20% or less, 10% or less, 5% or less, or 1% or less.

In one embodiment, the cell aggregate including dopamine-producing neuron progenitor cells and/or dopamine-producing neurons may comprise PAX6-positive cells in an amount of 5% or less, 2% or less, 1% or less, or 0.5% or less.

In one embodiment, the cell aggregate including dopamine-producing neuron progenitor cells and/or dopamine-producing neurons further comprises cells positive for TH and NURR1 in an amount of 20% or less, specifically 1% to 20%, more specifically 5% to 15%.

In one embodiment, the cell aggregate including dopamine-producing neuron progenitor cells and/or dopamine-producing neurons comprises cells positive for FOXA2 and LMX1A in an amount of 50% or more, preferably 60% or more, 70% or more, or 80% or more, and cells positive for TH and NURR1 in an amount of 20% or less, 1% to 20%, more specifically 5% to 15%.

In one embodiment, in the cell aggregate including dopamine-producing neuron progenitor cells and/or dopamine-producing neurons, SOX1-positive cells are 10% or less, preferably 7% or less, more preferably 3% or less, and PAX6-positive cells are 5% or less, preferably 4% or less, more preferably 2% or less.

In one embodiment, the cell aggregate including dopamine-producing neuron progenitor cells and/or dopamine-producing neurons comprises cells positive for FOXA2 and LMX1A in an amount of 60% or more and cells positive for TH and NURR1 in an amount of 1% to 20%, and SOX1-positive cells are 10% or less, preferably 7% or less, more preferably 3% or less, and PAX6-positive cells are 5% or less, preferably 4% or less, more preferably 2% or less.

In one embodiment, the cell aggregate including dopamine-producing neuron progenitor cells and/or dopamine-producing neurons comprises cells positive for FOXA2 and LMX1A in an amount of 60% or more of the total cells and may comprise cells positive for TH and NURR1 in an amount of 20% or less, 1 to 20%, or 5 to 15% of the total cells.

In one embodiment, the above cell aggregate including dopamine-producing neuron progenitor cells and/or dopamine-producing neurons is a cell aggregate having an equivalent spherical diameter of 150 µm to 1000 µm.

In one embodiment, the above cell aggregate including dopamine-producing neuron progenitor cells and/or dopamine-producing neurons comprises cells positive for FOXA2 and LMX1A in an amount of 60% or more and cells positive for NURR1 and TH in an amount of 1% to 20% and has an equivalent spherical diameter of 150 µm to 1000 µm.

### [Freezing method]

The method of the present application comprises the step of (1) contacting a cell aggregate including neural cells and having a three-dimensional structure with a preservation solution at 0°C to 30°C prior to freezing to prepare a preservation solution-soaked cell aggregate.

In the present application, a cryopreservation solution (preservation solution) refers to an aqueous solution comprising a cryoprotectant. The cryoprotectant refers to a substance that have a high affinity with water molecules and are highly effective in inhibiting the growth of ice crystals in the cryopreservation solution. Cryoprotectants include, for example, dimethyl sulfoxide (DMSO), ethylene glycol (EG), propylene glycol (PG), 1,2-propanediol (1,2-PD), 1, 3-propanediol (1,3-PD), butylene glycol (BG), isoprene glycol (IPG), dipropylene glycol (DPG), and/or glycerin. In the present application, the cryoprotectant is preferably dimethyl sulfoxide and/or propylene glycol. A concentration of the cryoprotectant in the cryopreservation solution is usually 7 to 12%, preferably about 10%, when dimethyl sulfoxide and/or propylene glycol is used as the cryoprotectant.

As the aqueous solution, for example, physiological saline, a buffered solution such as PBS, EBSS, or HBSS, a medium for culturing cells or tissues such as DMEM, GMEM, or RPMI, serum, a serum substitute, or a mixture thereof can be used.

As the cryopreservation solution, a commercially available cryopreservation solution comprising dimethyl sulfoxide (DMSO) and/or propylene glycol as a substantial component can be used. Specific examples of the cryopreservation solution include commercially available cryopreservation solutions such as STEM-CELL BANKER (SCB; ZENOAQ), STEM-CELL BANKER DMSO free (SCB DMSO free; ZENOAQ), Bambanker hRM (BBK; NIPPON Genetics), CryoStor CS5 (CS5; BioLife Solutions), CryoStor CS10 (CS10; BioLife Solutions), and Synth-a- Freeze (SaF; Thermo Fisher Scientific). For example, it is desirable to use a cryopreservation solution (such as STEM-CELL BANKER, Bambanker hRM, CryoStor CS10, or Synth-a-Freeze) comprising 7 to 12%, preferably about 10% of dimethyl sulfoxide and/or propylene glycol.

More preferably, Bambanker hRM can be used.

In the present specification, when the cell aggregate is frozen, number of cells relative to the cryopreservation solution (cell packing density) is 80000 to 5000000 cells/mL, 100000 to 4000000 cells/mL or 200000 to 2000000 cells/mL, 300000 to 1000000 cells/mL.

In the present specification, when the cell aggregate is frozen, the cell aggregate has an equivalent spherical diameter of 150 to 1000 µm, 150 µm to 600 µm, or 300 µm to 500 µm.

In the present specification, the cell aggregate and the preservation solution have volume of 0.25 mL to 2 mL, 0.5 mL to 1.5 mL, or 0.5 mL to 1 mL.

In the present specification, the cell aggregate and the preservation solution may be packed in a 0.5 mL to 15 mL, 1 mL to 5 mL, or 1 mL to 2 mL container.

The freezing point of the cryopreservation solution in the present application is not particularly limited but is usually -1°C to -10°C, preferably -3°C to -10°C, more preferably -3°C to -6°C, even more preferably about -5°C. Examples of the cryopreservation solution in the present specification include an aqueous solution comprising 7 to 12%, preferably about 10% of dimethyl sulfoxide as a substantial component and having the freezing point of - 1°C to -10°C. Also, examples of the cryopreservation solution in the present specification include an aqueous solution comprising 7 to 12%, preferably about 10% of dimethyl sulfoxide as a substantial component and having the freezing point of -3°C to -6°C.

The temperature at which the cell aggregate including neural cells is contacted with the cryopreservation solution is usually from 0°C to 30°C, preferably from 0°C to 20°C, more preferably from 0°C to 10°C, and even more preferably from 0°C to 4°C.

The period for which the cell aggregate including neural cells is contacted with the cryopreservation solution is usually 5 minutes to 240 minutes, 5 minutes to 120 minutes, preferably 5 minutes to 60 minutes, 15 minutes to 240 minutes, 15 minutes to 180 minutes, 15 minutes to 150 minutes, preferably 15 minutes to 120 minutes, 15 minutes to 90 minutes, and more preferably 15 minutes to 60 minutes.

The method of the present application also comprises the step of (2) cooling the preservation solution-soaked cell aggregate obtained in step (1) from a temperature at least about 5°C higher than the freezing point of the preservation solution to a temperature about 5°C lower than the freezing point at an average cooling speed of 2 to 7 °C/min, 2.5 to 7 °C/min, or 3 to 7 °C/min to freeze the cell aggregate.

In the method of the present application, the preservation solution-soaked cell aggregate is cooled from a temperature at least about 5°C higher than the freezing point of the preservation solution to a temperature about 5°C lower than the freezing point at an average cooling speed of 2 to 7 °C/min, 2.5 to 7 °C/min, or 3 to 7 °C/min, preferably 2 to 5.5 °C/min, 2.5 to 5.5 °C/min, or 3 to 5.5 °C/min. In a preferred embodiment, the preservation solution-soaked cell aggregate is cooled from 0 ± 5°C to - 30°C ± 5°C at an average cooling speed of 2 to 5 °C/min, 2.5 to 5 °C/min, or 3 to 5 °C/min.

The means for cooling is not particularly limited as long as the above step is achieved; a commercially available freezer can be used, and a programmed freezer (also referred to as a controlled-rate freezer) capable of controlling the temperature may be used.

In step (2), the preservation solution-soaked cell aggregate may be exposed to an electromagnetic field and/or a magnetic field. The electromagnetic field has a frequency of, for example, about 300 kHz to about 2 MHz, preferably about 500 kHz to about 1 MHz, more preferably about 600 kHz to about 1 MHz. A frequency of the magnetic field is not particularly limited but is preferably a fixed frequency. In addition, the freezing can be performed under an electrostatic field of, for example, 10 to 2000 Gauss, preferably 50 to 1000 Gauss, and more preferably 100 to 150 Gauss.

A method for achieving the above condition is not particularly limited, and for example, a programmed freezer equipped with a device for generating an electromagnetic field and a magnetic field, which is commercially available as a proton freezer (RYOHO FREEZE SYSTEMS. CO., LTD.), can be used. That is, a device having a combination of a magnetic field (a static magnetic field (SMF)), an electromagnetic wave (an alternating electric field (AEF)), and ultra-cold air may be used. More specifically, use of an electromagnetic wave of 300 kHz to 2 Mhz can inhibit the cell aggregate from being injured by formation of ice.

The method of the present application may further comprise the step of (3) cooling the frozen cell aggregate obtained in step (2) to -50°C or lower, preferably -80°C or lower, more preferably -150°C or lower.

The means for cooling is not particularly limited, and examples thereof include deep freezers, programmed freezers, proton freezers, and use of a low-temperature medium (e.g., liquid nitrogen).

The frozen cell aggregate obtained in step (2) or (3) may be held at -80°C or lower, preferably -150°C or lower for long-term preservation.

Means for long-term preservation is not particularly limited, and examples thereof include deep freezers, programmed freezers, proton freezers, and use of a low-temperature medium (e.g., liquid nitrogen).

The frozen cell aggregate can be thawed and used as appropriate. The thawing method is not particularly limited, but it is desirable to perform the thawing at about body temperature in a short period from the viewpoint of the function, activity, and viability of the cells. Specifically, it is desirable to perform the thawing at 30°C to 40°C, preferably at 35°C to 38°C, and more preferably at a temperature around human body temperature, for example, at about 37°C.

The cell aggregate frozen by the method of the present invention may be subjected to recovery culture after thawing by replacing the cryopreservation solution with a medium or may be transplanted into a living body without performing recovery culture.

That is, the cell aggregate frozen by the method of the present invention can maintain properties equivalent to those of an unfrozen cell aggregate. For example, the cell aggregate frozen by the method of the present invention and then subjected to recovery culture for 7 days after thawing has a marker expression rate equivalent to that of the cell aggregate before freezing. For example, in the case of the cell aggregate including dopamine-producing neuron progenitor cells, examples of markers expressed on the cells include FOXA2, LMX1A, NURR1 and TH. The equivalent marker expression rate means that the difference in numerical values of the percentages of the cells expressing a maker to the total cells between before freezing and after thawing, or after culturing for 7 days after thawing is about 10% or less.

The cell aggregate frozen by the method of the present invention is useful in that it can be transplanted into a living body without recovery culture.

### [Pharmaceutical composition]

The present application further provides a pharmaceutical composition, i.e., a composition (formulation) for transplantation, comprising, as an active ingredient, the cell aggregate frozen or preserved for a long-term by the above method.

The pharmaceutical composition (composition for transplantation) of the present invention is a concept including both a pharmaceutical composition frozen by the method of the present invention and a pharmaceutical composition obtained by thawing the frozen pharmaceutical composition. That is, examples of the pharmaceutical composition (composition for transplantation) of the present invention include a frozen or unfrozen composition comprising the cell aggregate including neural cells and the cryopreservation solution, as well as a composition comprising the cell aggregate including neural cells and a dosing vehicle where the cryopreservation solution has been replaced with the dosing vehicle after thawing.

Examples of the pharmaceutical composition (composition for transplantation) of the present invention include the compositions for transplantation according to [20] to [27] above.

Also, in one embodiment, the present application provides a frozen composition for transplantation, wherein the composition comprises:
a cell aggregate including 60% or more of dopamine-producing neuron progenitor cells and dopamine-producing neurons derived from a pluripotent stem cell, having an equivalent spherical diameter of 150 µm to 1000 µm and including 500 to 150000 cells; and
   a cryopreservation solution comprising 7% to 12% of dimethyl sulfoxide or propylene glycol and having the freezing point of -1°C to -10°C, the cryopreservation solution being preferably Bambanker hRM, and
wherein the frozen cell aggregate has following properties:
   (1) about 60% or more of the total cells are viable after thawing;
   (2) the cells after thawing have a neurite extension activity of 50% or more of that before freezing; and
   (3) the rate of the FOXA2-positive cells, LMX1A-positive cells, NURR1-positive cells, and TH-positive cells in the cells viable after thawing are changed within ± 10% from those in the cells before freezing, preferably, the rate of the FOXA2-positive cells, LMX1A-positive cells, NURR1-positive cells, TH-positive cells, EN1-positive cells, and PITX3-positive cells are changed within ± 10%.

As one embodiment, the present invention encompasses a composition for transplantation wherein number of cells in the composition is 80000 to 5000000 cells/mL, 100000 to 4000000 cells/mL, or 200000 to 2000000 cells/mL, 300000 to 1000000 cells/mL, and the composition comprises FOXA2-positive and LMX1A-positive cells in an amount of 40% or more, preferably 60% or more, 60% or more, 80% or more, 85% or more, or 90% or more of the total cells, and TH-positive and NURR1-positive cells in an amount of 40% or less, 1% to 20%, or 5% to 15% of the total cells.

In one embodiment, the cell aggregate has an equivalent spherical diameter of 150 to 1000 µm, 150 µm to 600 µm, or 300 µm to 500 µm.

In one embodiment, the cell aggregate and the preservation solution have volume of 0.25 mL to 2 mL, 0.5 mL to 1.5 mL, or 0.5 mL to 1 mL.

In one embodiment, the cell aggregate and the preservation solution may be packed in a 0.5 mL to 15 mL, 0.5 mL to 5 mL, or 1 mL to 2 mL container.

As one embodiment, the present invention encompasses a composition for transplantation that can be used without recovery culture after thawing.

As one embodiment, the present invention encompasses the composition for transplantation according to any one of [20] to [24], wherein the composition comprises the cell aggregates in 8 to 192 cell aggregates/mL, the cell aggregates have an average particle size of 150 µm to 1000 µm, and number of cells per container is 80000 to 2400000.

The cell aggregate is useful as a pharmaceutical composition for transplantation for a patient suffering from a disease requiring transplantation of neural cells and can be used as a drug such as a therapeutic drug for a disease accompanied by degeneration, injury, or dysfunction of neural cells. That is, a pharmaceutical composition comprising the cell aggregate of the present invention and a pharmaceutically acceptable carrier is also within the scope of the present invention.

Examples of the disease requiring transplantation of neural cells or the disease accompanied by injury or dysfunction of neural cells include spinal cord injury, motor nerve disease, multiple sclerosis, amyotrophic lateral sclerosis, atrophic lateral sclerosis, Huntington's disease, multiple system atrophy, spinocerebellar degeneration, Alzheimer's disease, Retinitis pigmentosa, age-related macular degeneration, and parkinsonian syndrome (including Parkinson's disease).

One embodiment of the present invention includes a pharmaceutical composition for treating Parkinson's disease, comprising, as an active ingredient, the cell aggregate of the present invention including dopamine-producing neuron progenitor cells and/or dopamine-producing neurons. Number of dopamine-producing neuron progenitor cells and/or dopamine-producing neurons included in the therapeutic agent for Parkinson's disease is not particularly limited as long as the graft can be engrafted after the administration; for example, 1.0 × 10⁴ cells or more can be included per transplantation. In addition, number of cells may be appropriately increased or decreased according to symptoms or body size to prepare the therapeutic agent. Transplantation of the dopamine-producing neuron progenitor cells to a disease site can be performed, for example, by the technique described in Nature Neuroscience, 2, 1137 (1999) or N Engl J Med.; 344: 710 to 9 (2001).

As one embodiment, the pharmaceutical composition (also referred to as the composition for transplantation) of the present invention comprises a cell aggregate including neural cells to be transplanted into a human and a cryopreservation solution. The pharmaceutical composition of the present invention includes both a frozen solid form and a liquid form before freezing or after thawing. The pharmaceutical composition may include an additive used to maintain the survival of cells as appropriate, to such an extent that it will not affect the freezing rate and freezing temperature. Examples of the cryopreservation solution include those described above.

As described below, the pharmaceutical composition or the composition for transplantation of the present invention is used for transplantation after thawing it and removing and replacing the cryopreservation solution with a dosing vehicle injectable to a living body. That is, a composition comprising the thawed cell aggregate and the dosing vehicle is also within the scope of the pharmaceutical composition (also referred to as the composition for transplantation) of the present invention.

### [A method for producing a composition for transplantation]

The pharmaceutical composition (composition for transplantation) according to [20] to [27] above can be produced by the freezing method according to any one of [1] to [17] above. That is, the present invention encompasses a method for producing the above-described pharmaceutical composition (composition for transplantation).

### [Therapeutic method]

One embodiment of the present invention includes a method for treating a disease requiring supplementation of neural cells, which comprises the step of transplanting the cell aggregate of the present invention into a patient suffering from the disease requiring transplantation of neural cells.

In one embodiment of the present invention, the cell aggregate including dopamine-producing neuron progenitor cells and/or dopamine-producing neurons as obtained by the present invention can be administered to a patient with Parkinson's disease as a pharmaceutical composition, specifically as material for transplantation.

Specifically, the administration is performed as follows: the frozen pharmaceutical composition of the present invention comprising the cell aggregate including dopamine-producing neuron progenitor cells and/or dopamine-producing neurons, and the cryopreservation solution is thawed, appropriately suspended in an appropriate medium for transplantation such as physiological saline, and then transplanted into a region of a patient where dopaminergic neurons are insufficient, for example, the corpus striatum. For example, the pharmaceutical composition after thawing may be washed with a vehicle comprising an appropriate carrier, and the cryopreservation solution may be replaced with a transplantation vehicle for suspending the cell aggregate for the transplantation into a human. A thawing temperature is not particularly limited but can be 30°C to 40°C, preferably 35°C to 38°C, and more preferably a temperature around human body temperature, for example, about 37°C as described above.

The cell aggregate included in the pharmaceutical composition (composition for transplantation) of the present invention can be transplanted into a living body by replacing the cryopreservation solution with a dosing vehicle without recovery culture after thawing.

For the carrier used for the transplantation vehicle (dosing vehicle) used for the cell aggregate including dopamine-producing neuron progenitor cells and/or dopamine-producing neurons, any substance known to those skilled in the art can be used, as long as it is a substance used to maintain survival of cells. Specifically, a physiological aqueous solvent (such as physiological saline, a buffer solution, serum-free medium, etc.) can be used. In transplantation medicine, if necessary, the pharmaceutical composition including tissue or cells to be transplanted may be combined with a preservative, a stabilizing agent, a reducing agent, or an isotonizing agent which is commonly used.

For the transplantation, the thawed cell aggregate may be preserved in a vehicle necessary to maintain the viability of each cell aggregate. Examples of the "vehicle necessary to maintain the viability" include medium and a physiological buffer solution; the vehicle is not particularly limited as long as the vehicle allows the cell population including dopamine-producing neuron progenitor cells and/or dopamine-producing neurons to survive, and those skilled in the art can appropriately select such a vehicle. One example of the vehicle is a medium prepared by using a medium usually used for culturing animal cells as a basal medium. Examples of the basal medium include media that can be used for culturing animal cells, such as BME medium, BGJb medium, CMRL 1066 medium, GMEM medium, Improved MEM Zinc Option medium, Neurobasal medium, IMDM medium, Medium 199 medium, Eagle MEM medium, αMEM medium, DMEM medium, F-12 medium, DMEM/F12 medium, IMDM/F12 medium, Ham medium, RPMI 1640 medium, Fischer's medium or mixed media thereof.

Due to the transplantation of the above cell aggregate, the transplanted dopamine-producing neuron progenitor cells and/or dopamine-producing neurons as well as dopamine-producing neuron progenitor cells and/or dopamine-producing neurons that are induced after the transplantation are functionally engrafted in the patient to which the cell aggregate is administered.

The term "engraftment" as used herein means that transplanted cells survive in a living body for a long period of time (e.g., 30 days or more, 60 days or more, or 90 days or more) and adhere and remain in an organ.

The term "functional engraftment" as used herein means a state in which transplanted cells are engrafted to perform their original function in a living body.

The term "functional survival rate" as used herein refers to the percentage of the cells that have achieved functional engraftment in the transplanted cells. The functional survival rate of transplanted dopamine-producing neuron progenitor cells can be determined, for example, by measuring number of TH-positive cells in the graft.

Due to the transplantation of the above cell aggregate, the transplanted cells and the dopamine-producing neuron progenitor cells and/or dopamine-producing neurons induced after the transplantation have a functional survival rate of 0.1% or more, preferably 0.2% or more, more preferably 0.4% or more, even more preferably 0.5% or more, and still more preferably 0.6% or more.

As one embodiment, the present invention encompasses a method for treating a disease requiring regeneration of a dopamine-producing nerve, which comprises following steps of:
(1) thawing the composition for transplantation according to any one of [20] to [27] at 30°C to 40°C, preferably at 37°C ± 3°C; and
(2) transplanting the composition for transplantation obtained in (1) into a corpus striatum region of a patient.

One embodiment of the present invention encompasses the treating method, wherein the cryopreservation solution is replaced with a dosing vehicle without culture after thawing to perform step (2).

Examples of a mammal to undergo the transplantation in the present specification include humans, mice, rats, guinea pigs, hamsters, rabbits, cats, dogs, sheep, pigs, cows, horses, goats, and monkeys, preferably rodents (e.g., mice and rats) or primates (e.g., humans and monkeys), and more preferably humans.

### EXAMPLES

Hereinafter, the present application is described in more detail with reference to Examples but is not limited to the Examples.

### Example 1

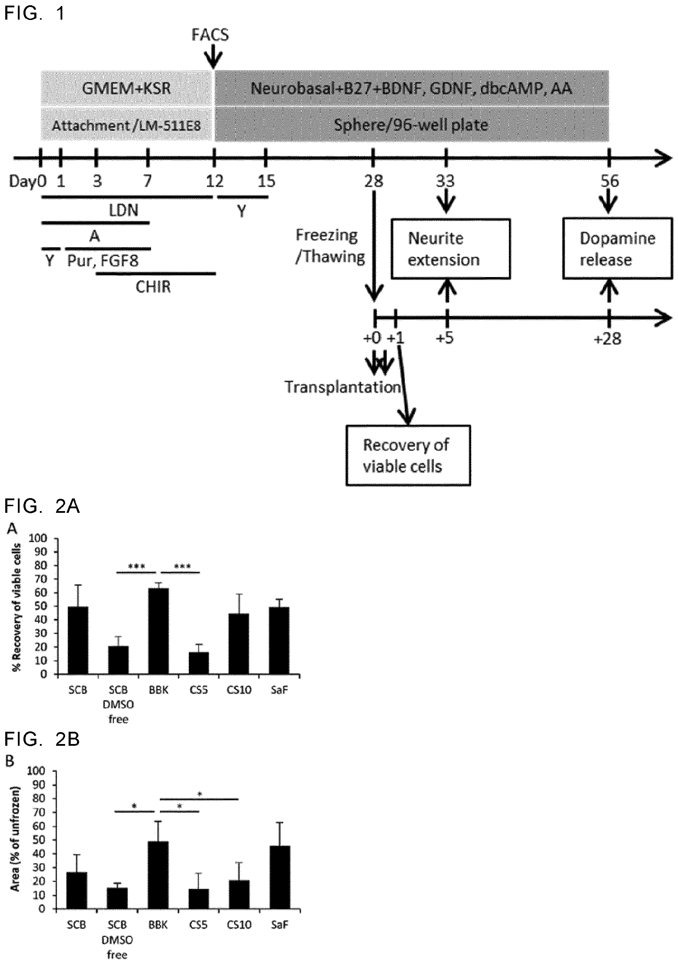

An outline of Example 1 is shown in Figure 1.

### [Material and method]

### Maintenance and neural differentiation of human iPS cells

In accordance with the dual SMAD inhibition and floor plate induction protocol described in Doi et al., 2014, iPSC-derived dopamine-producing neuron progenitor cells were inducted. That is, human iPSCs (1231A3) (Kyoto University) were maintained in Stem Fit medium (AJINOMOTO CO., INC.) on a 6-well plate coated with iMatrix-511 (Nippi, Inc.). To initiate neuronal differentiation, the iPSCs were incubated with TrypLE select (Invitrogen) for 10 minutes, then dissociated into single cells and seeded with differentiation medium at a density of 5 × 10⁶ cells/well on the 6-well plate coated with iMatrix-511 (Nippi, Inc.). The differentiation medium was medium containing GMEM supplemented with 8% KSR, 0.1 mM MEM non-essential amino acids (all from Invitrogen), sodium pyruvate (Sigma-Aldrich), and 0.1 mM 2-mercaptoethanol. The differentiation medium was changed every day from the day after seeding until day 12. To increase cell viability after seeding, 10 µM Y-27632 (WAKO CHEMICAL CO., LTD.) was added on day 1. To efficiently induce the neuronal differentiation, LDN193189 (STEMGENT, INC.) and A83-01 (WAKO CHEMICAL CO., LTD.) were added. To induce floor plate cells, 2 µM Purmorphamine and 100 ng mL⁻¹ FGF8 (WAKO CHEMICAL CO., LTD.) were added on days 1 to 7, and 3 µM CHIR99021 (WAKO CHEMICAL CO., LTD.) was added on days 3 to 12 (see Figure 1).

### Cell sorting and culture

On day 12, cells expressing CORIN (a floor plate marker in the developing brain) were isolated to concentrate the dopamine-producing neuron progenitor cells, and sorting (FACS technique) was performed thereon. First, cultured cells were stained with PE-labeled anti-CORIN antibody (100ng/mL; Catalent/BD) for 20 minutes. Dead cells and debris were excluded through 7-AAD staining. Analysis was performed using a FACSAria II or III cell sorter and FACSDiva software program (BD Biosciences). After cell sorting on day 12 of culture, the sorted cells were reseeded at a density of 2-3 × 10⁴ cells/well in neuronal differentiation medium containing neurobasal medium supplemented with B27 supplement, 2 µM Glutamax-I (all from Invitrogen), 10 ng mL⁻¹ GDNF, 200 mM ascorbic acid, 20 ng mL⁻¹ BDNF (all from WAKO CHEMICAL CO., LTD.) and 400 µM dbcAMP (Sigma-Aldrich) on a low cell adhesion U-shaped 96-well plate (Sumitomo Bakelite Co., Ltd.), and cultured as floating spheres until day 28. Unsorted cells were also reseeded at a density of 1.5 × 10⁴ cells/well. Half of the medium was replaced every 3 days, and 30 µM Y-27632 (WAKO CHEMICAL CO., LTD.) was added only to the first medium. For prolonged culture, the floating spheres were cultured in the neuronal differentiation medium (see Figure 1).

### Cryopreservation method

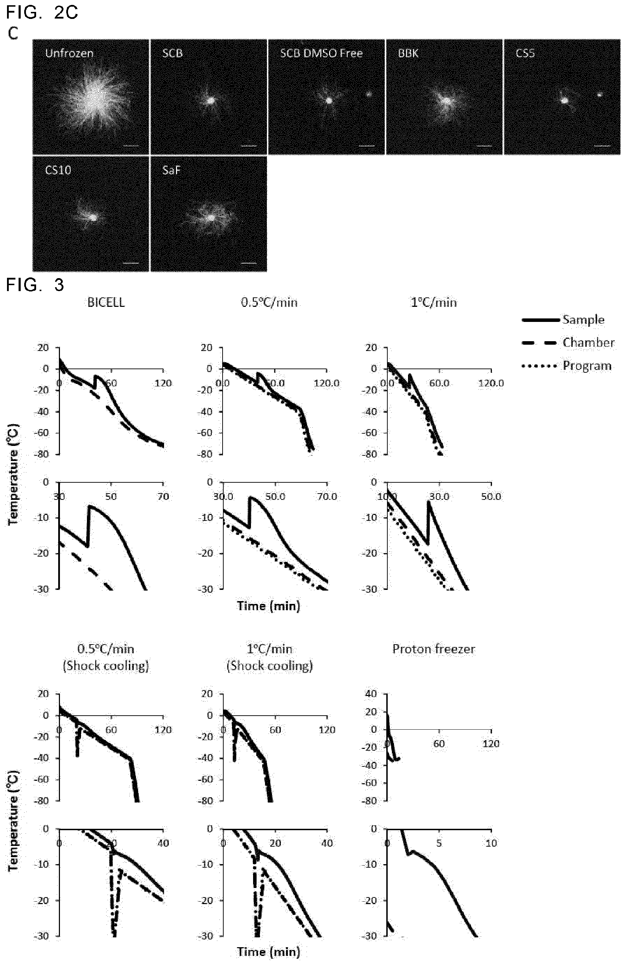

The spheres collected on day 28 were cryopreserved and used in subsequent experiments. That is, the collected spheres were placed in cryovials containing 1 mL of ice-cold cryopreservation solution shown in Table 1 and kept on ice until freezing. For cryopreservation, the vials were transferred into a freezing container: BICELL (NIHON FREEZER CO., LTD.), a programmed freezer: PDF-150, 250 (STREX Inc.), cryomed (Thermo Fisher Scientific Inc.) or a proton freezer (RYOHO FREEZE SYSTEMS. CO., LTD.). Six different cooling profiles (shown in Figure 3) were used in this experiment. That is, BICELL was transferred into a deep freezer (-80°C) and kept for 4 hours or more (upper left in Figure 3). In the controlled-rate freezing method, the vials were frozen to -40°C at a rate of 0.5 °C/min (upper center in Figure 3) or 1 °C/min (upper right in Figure 3) and then cooled to -80°C at a faster rate of 3 to 5 °C/min. In the shock cooling method, the procedure of freezing to -35°C at a rate of -25°C/min and subsequent warming to -12°C at a rate of +10°C/min was inserted at a temperature of -4°C during freezing (lower left and center in Figure 3). The vials frozen with the proton freezer were kept in a chamber for 30 to 60 minutes (lower right in Figure 3). The proton freezer has a static magnetic field, electromagnetic waves, and cold air in combination. It is considered that static magnetic fields and electromagnetic fields influence the orientation of water molecules and cause the formation of small ice crystals, thereby preventing cell disruption. However, these mechanisms have not yet been fully understood. After freezing, the cryovials were preserved in the vapor phase of a liquid nitrogen tank. The frozen cells were thawed at 37°C for about 2 minutes and transferred into a 15 mL tube containing 10 mL of neurobasal medium. After removal of the supernatant, the cells were rinsed with PBS and used for each assay or transplantation. To estimate number of cells after cryopreservation, about 50 aggregates were dissociated and counted with a hemacytometer to calculate the cell concentration one day after thawing and before freezing.

Table 1 Commercially available xeno-free cryopreservation solutions used in the present Examples

**[Table 1]**

| Medium | Brand name | Supplier | Mejor component |
|---|---|---|---|
| SCB | STEM-CELLBANKER | ZENOAQ | 10 % DMSO |
| SCB DMSO free | STEM-CELLBANKER DMSO free | ZENOAQ | 10 % Propylene glycol |
| BBK | Bambanker hRM | NIPPON Genetics | 10 % DMSO |
| CS5 | Cryostor CS5 | BioLife Solutions | 5 % DMSO |
| CS10 | Cryostor CS10 | BioLife Solutions | 10 % DMSO |
| SaF | Synth-a-Freeze | Thermo Fisher Scientific | 10 % DMSO |

### Quantitative RT-PCR

Total RNA was extracted from undifferentiated cells, iPSC-derived dopamine-producing neurons (on day 28, day 29, day 31, and day 35), and iPSC-derived dopamine-producing neurons after thawing on days 28 + 0, 1, 3, and 7 using RNeasy Mini Kit or RNeasy Micro Kit (Qiagen) and cDNA was synthesized using Super Script III First-Strand Synthesis System (Invitrogen). Quantitative PCR reactions were performed by using Power SYBR Green PCR Master Mix (Applied Biosystems) with StepOne. Data were evaluated using the delta Ct method and normalized by GAPDH expression. The primer sequences used are shown in Table 2.

**[Table 2]**

| **Gene** | **Forward** | **Reverse** |
|---|---|---|
| POU5F1 | AGACCATCTGCCGCTTTGAG (SEQ ID NO: 1) | GCAAGGGCCGCAGCTT (SEQ ID NO: 2) |
| NANOG | GGCTCTGTTTTGCTATATCCCCTAA (SEQ ID NO: 3) | CATTACGATGCAGCAAATACGAGA (SEQ ID NO: 4) |
| FOXA2 | TTCAGGCCCGGCTAACTCT (SEQ ID NO: 5) | AGTCTCGACCCCCACTTG CT (SEQ ID NO: 6) |
| LMX1A | GATCCCTTCCGACAG GGTCTC (SEQ ID NO: 7) | GGTTTCCCACTCTGGACTGC (SEQ ID NO: 8) |
| EN1 | TGGGTGTACTGCACACGTTATTC (SEQ ID NO: 9) | GGAACTCCGCCTTGAGTCTCT (SEQ ID NO: 10) |
| NURR1 | CGAAACCGAAGAGCCCACAGGA (SEQ ID NO: 11) | GGTCATAGCCGGGTTGGAGTCG (SEQ ID NO: 12) |
| PITX3 | GGGCCAGGAGCACAGCGACTCA (SEQ ID NO: 13) | GCTGCCGCCGCTGCTTCTTTTT (SEQ ID NO: 14) |
| TH | GCAGTTCTCGCAGGACATTG (SEQ ID NO: 15) | CGGCACCATAGGCCTTCA (SEQ ID NO: 16) |
| TPH2 | TCAGCTACTTGGCAGCTCAAC (SEQ ID NO: 17) | CTTGCCACTTTCGGTAGCAG (SEQ ID NO: 18) |
| GAPDH | GGTCGGAGTCAACGGATTTG (SEQ ID NO: 19) | TCAGCCTTGACGGTGCCATG (SEQ ID NO: 20) |

### Immunofluorescence studies

In *in vitro* studies, cultured cells on day 35 or on day 7 after thawing were fixed with 4% paraformaldehyde. In the *in vivo* studies, fixed and frozen brains were sliced into 40-µm thin slices. The slices were immunostained using the free-floating method. The primary antibodies used are shown in Table 3. Cells were visualized using a fluorescence microscope (BZ-9000; Keyence Corporation) and a confocal laser scanning microscope (Fluoview FV1000D; Olympus). Number of immunoreactive cells was quantified in every 6th sectionthroughout the grafts and corrected using the Abercrombie method.

**[Table 3]**

| **Antibody** | **Species** | **Dilution** | **Supplier** | **Catalog#** |
|---|---|---|---|---|
| TH | Rabbit | 1:400 | Millipore | AB152 |
| NURR1 | Rat | 1:1000 | Donated by KAN labora tory | - |
| FOXA2 | Goat | 1:500 | R&D systems | AH2400 |
| SOX1 | Goat | 1:100 | R&D systems | AH3369 |
| PAX6 | Mouse | 1:500 | BD Pharmingen | 561462 |
| KI67 | Rabbit | 1:1000 | Novocastra | NCLKi67P |
| TUBB3 | Mouse | 1:400 | Covance | MMS-435P |
| HNA | Mouse | 1:500 | Millipore | MAB1281 |

| | | | | |
|---|---|---|---|---|
| *TUBB3 indicates βIII-Tubulin. | | | | |

### Neurite extension assay

On day 28, floating spheres picked for a neurite extension assay were cultured on a 24-well plate coated with iMatrix-511 for 5 days and fixed with 4% paraformaldehyde. The spheres were stained with PE-labeled anti-PSA-NCAM antibody (1:100; Miltenyi Biotec) and visualized using a fluorescence microscope (BZ-9000; Keyence). The area of PSA-NCAM-positive neurites without cell bodies was measured using Photoshop (Adobe systems) and WinRoof (Mitani Corporation).

### Electrophysiologic analysis

The suspension spheres were dissociated with papain on day 28 and cultured on a plate coated with poly-l-ornithine, fibronectin, and laminin (O/F/L) until day 49. Nerves with large cell bodies and neurite-like structures were selected for whole-cell patch-clamp recording. The cells were maintained in a physiological saline solution containing following components: 125 mM NaCl, 2.5 mM KCl, 2 mM CaCl₂, 1 mM MgCl₂, 26 mM NaHCO₃, 1.25 mM NaH₂PO₄, and 17 mM glucose. A patch pipette was prepared from a borosilicate glass capillary (GC150TF-10; Clark). This patch pipette had a resistance of 3 to 4 MW when filled with an internal solution composed of 140 mM KCl, 10 mM HEPES, and 0.2 mM EGTA (pH 7.3). Voltage-clamp and current-clamp recordings were performed using a patch-clamp amplifier (EPC-8; HEKA). The gigaseal resistances were in the range of 10 to 20 GW. The current signals from a patch clamp amplifier were filtered at 5 kHz through a 4-pole low-pass filter (UF-BL2; NF) having the Bessel characteristics, sampled using a 12-bit A/D converter, and stored on a 32-bit computer (PC-9821Ra333; NEC). All experiments were performed at room temperature.

### Dopamine release assay

On day 28, floating spheres picked for a dopamine release assay were cultured on a 12-well plate coated with O/F/L for another 28 days, washed twice with a solution having a low concentration of KCl (4.7 mM), and incubated in a solution having a low concentration of KCl for 15 minutes. The medium was then replaced with a solution having a high concentration of KCl (60 mM) for 15 minutes. The solution was collected, and its dopamine concentration was determined by LC/MS/MS. The cells remaining on the plate were collected in PBS and sonicated. The DNA concentration of a cell lysate was measured using the Quant-iT ^{™} dsDNA Assay Kit (Thermo Fisher Scientific Inc.) and used to correct the dopamine concentration.

### Microarray analysis

A cDNA microarray analysis was performed in the BioMedical Department of Kurabo Industries Ltd. The total RNA of the undifferentiated cells, the cultured cell on day 12, the iPSC-derived dopamine-producing neurons (day 28, day 29, day 31, and day 35), and the iPSC-derived dopamine-producing neurons after thawing on days 28 + 0, 1, 3, and 7 was processed using Genechip^{®} 3'IVT pico Reagent Kit and Human Genome U133 Plus 2.0 array (Affymetrix, Inc.). Data analysis was performed using Genechip Operating Software ver. 1.4 (Affymetrix, Inc.). Signal detection and quantification was performed using MAS5 algorithm. Global normalization was performed such that the average signal intensity of all probe sets was equal to 100. Analysis was performed using data on a probe set showing a signal intensity higher than 50 at p < 0.05 and showing a 2-fold or more change between the samples.

### Cell transplantation

Experimental animals were treated and handled in accordance with the Guidelines for Animal Experiments of Sumitomo Dainippon Pharma Co., Ltd., Guidelines for Animal Experiments of Kyoto University, and the Guide for the Care and Use of Laboratory Animals of the Institute of Laboratory Animal Resources (ILAR; Washington, DCA). Male F344/NJcl-rnu/rnu (nude) rats, Parkinson's disease model F344 NJcl-rnu/rnu rats (CLEA Japan, Inc.), and Sprague Dawley (SD) rats (SHIMIZU LABORATORY SUPPLIES Co., Ltd.) were used for a short-term transplantation study. The SD rats were injected with 6-OHDA into the medial forebrain bundle in the right hemisphere at following coordinates (A, -4.0; L, -1.3; V, -7.0). A total of 19.2 mg of 6-OHDA (in 3 µL of physiological saline containing 0.02% ascorbic acid) was injected into each rat. From one day prior to transplantation, the SD rats were immunosuppressed daily with cyclosporine (10 mg/kg, i.p., LC Laboratories). Cell transplantation was performed with stereotactic injection of the spheres (A, +1.0; L, -3.0; V, -5.0 and -4.0; as well as TB, 0 (2 µl; 200,000 cells/µl).

Nude rat models for Parkinson's disease were used for a long-term transplantation study. Cell transplantation was performed with stereotactic injection of the unfrozen spheres (A, +1.0; L, -3.0; V, -5.0 and - 4.0; as well as TB, 0 (2µl; 200,000 cells/µl) or the cryopreserved spheres (A, +1.0; L, -3.5 and 2.5; V, -5.5 and -4.5; as well as TB, 0 (4µl; 200,000 cells/µl) into the corpus striatum of the right brain. The experimental animals were transcardially anesthetized and perfused with PBS followed by 4% paraformaldehyde.

### Behavioral analysis

An evaluation of a methamphetamine-induced rotational movement was performed before the transplantation and every 4 weeks after the transplantation using rotation bowls monitored with a video. Methamphetamine (Sumitomo Dainippon Pharma Co., Ltd.) at a dose of 2.5 mg/kg was injected intraperitoneally and rotations were recorded for 90 minutes.

### Statistical analysis

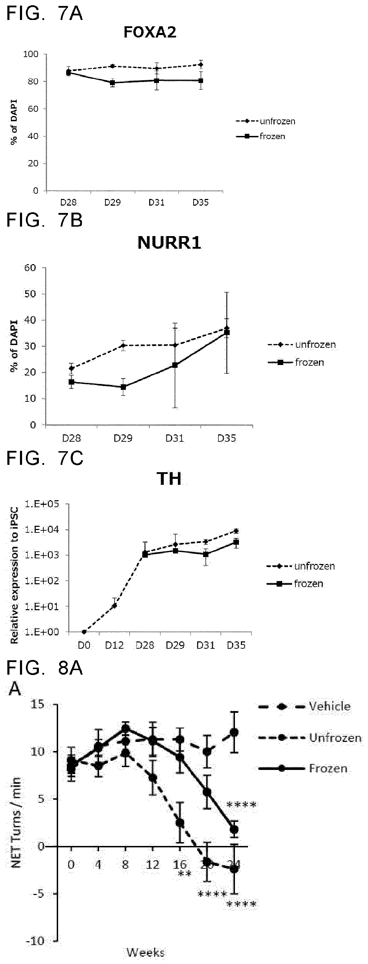

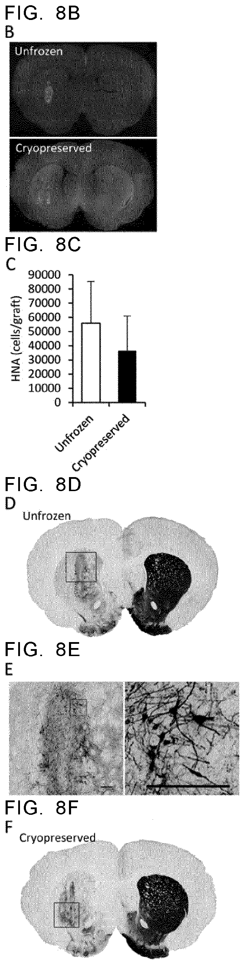

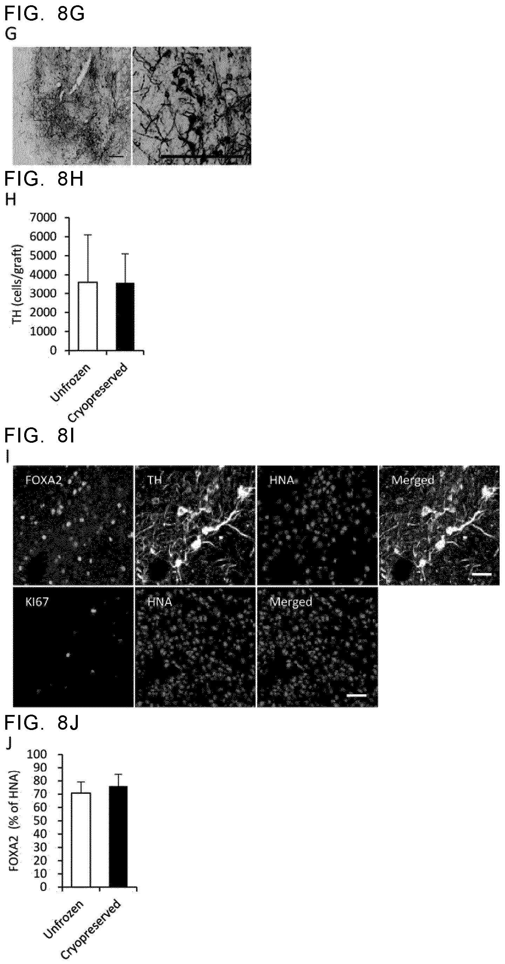

Statistical analysis was performed using a commercially-available software package (GraphPad Prism 6; GraphPad Software). Data from *in vitro* were analyzed by the one-way ANOVA and Tukey's post hoc analysis (Figures 2A, 2B, 4A, 4B, 5A, and 5B). Behavioral data were analyzed by two-way ANOVA with Tukey's multiple comparison test (Figure 8A). The differences were considered to be statistically significant for values of p < 0.05. The data were presented as mean ± SD, except for the behavioral data (mean ± SEM).

### [Result]

### (1) Examination result on cryopreservation solutions (Reference Example)

To establish the cryopreservation conditions suitable for iPSC-derived dopamine-producing neuron progenitor spheres as described above, clinically applicable cryopreservation solutions listed in Table 1 above were compared.

First, as described in Doi et al., 2014, conditions for a cryopreservation solution were screened using neural progenitor spheres that were not sorted by CORIN. It has been reported that number of cells reduces after thawing due to necrosis and subsequent persistence of delayed apoptosis and delayed necrosis (Milosevic et al., 2005). It is known that the proliferative capacity begins to recover during this process and number of cells bottoms out about 24 to 48 hours after thawing (Malpique et al., 2010; Mitchell et al., 2014; Baust et al., 2017). It has also been reported that neurotoxic compounds or diseases related to genetic aberration can cause neurite abnormality (Radio et al., 2008; Reinhardt et al., 2013; Ryan et al., 2016). These phenomena suggest that neurite morphology is a good indicator of characteristics of a neuron. Therefore, the recovery rate of viable cells one day after thawing was evaluated as cell viability, and also the area of neurite extension was examined as a new functional evaluation. The results are shown in Figure 2A, Figure 2B, and Figure 2C.

In all cryopreservation solutions, the cell viability and neurite extension significantly reduced after freezing (Figure 2). All the preservation solutions with 10% DMSO showed significantly higher viability than STEM-CELL BANKER DMSO free (SCB DMSO free) (21 ± 7%) and CryoStor CS5 (CS5) (16 ± 6%) (Figure 2A). Bambanker hRM (BBK) and Synth-a-Freeze (SaF) showed a tendency of relatively greater neurite extension than the other freezing conditions (Figure 2B and Figure 2C). 8 ± 17%, 50 ± 43%, and 13 ± 25% of the spheres did not adhere to the plate in the SCB DMSO free condition, CS5 condition, and CryoStor CS10 condition, respectively. Since BBK was already registered in the Drug Master File in Japan, BBK was used in following experiments.

### (2) Effects of intermediate speed cooling and suppression of supercooling

The unsorted spheres in BBK were then cryopreserved using six different protocols. The temperature profile for each protocol is shown in Figure 3. In addition, a shock cooling method having a process of transient temperature drop (Morris and Acton., 2013) was also examined in order to verify the effect of controlling ice nucleation and latent heat at the freezing point. Proton freezers have the distinctive cooling profile in addition. to magnetic field and electromagnetic waves. Proton freezers cool a sample from -4°C to -30°C at about 5°C/min without supercooling. This cooling rate is intermediate between the conventional slow cooling method and the vitrification method. Specifically, in the case of cooling in a proton freezer, the sample in the chamber is cooled at about 2 to 7°C/min as an actual value.

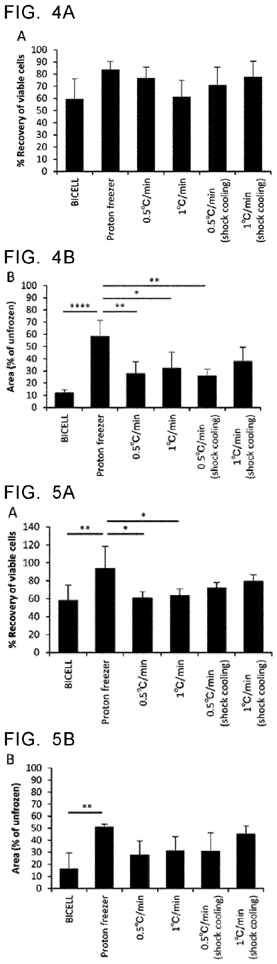

The conditions of the proton freezer and 0.5°C/min and 1°C/min without shock cooling process resulted in relatively higher cell viability than the other conditions (Figure 4A). In particular, the conditions of the proton freezer and 0.5°C/min showed about 80% cell viability compared to unfrozen cells. Also, the proton freezer showed significantly greater neurite extension than the other freezing conditions, except for the condition of 1°C/min with the shock cooling process, which was about 60% extension compared to that of the unfrozen cells (Figure 4B). These results demonstrate the maintenance of cellular functions in the condition of the proton freezer. When the time for immersion in the cryoprotectant before freezing was extended to 1 hour, good results for cell viability and neurite extension was obtained under the proton freezer condition as in the case of the immersion time of 15 minutes (Figures 5A and B). In addition, the cell viability was reduced under the condition of 0.5°C/min, but no significant differences were observed for cell viability or neurite extension under the other conditions (Figures 5A and 5B). Therefore, it was found that the intermediate speed cooling and suppression of supercooling are effective for cryopreservation of iPSC-derived dopamine-producing neuron progenitor spheres.

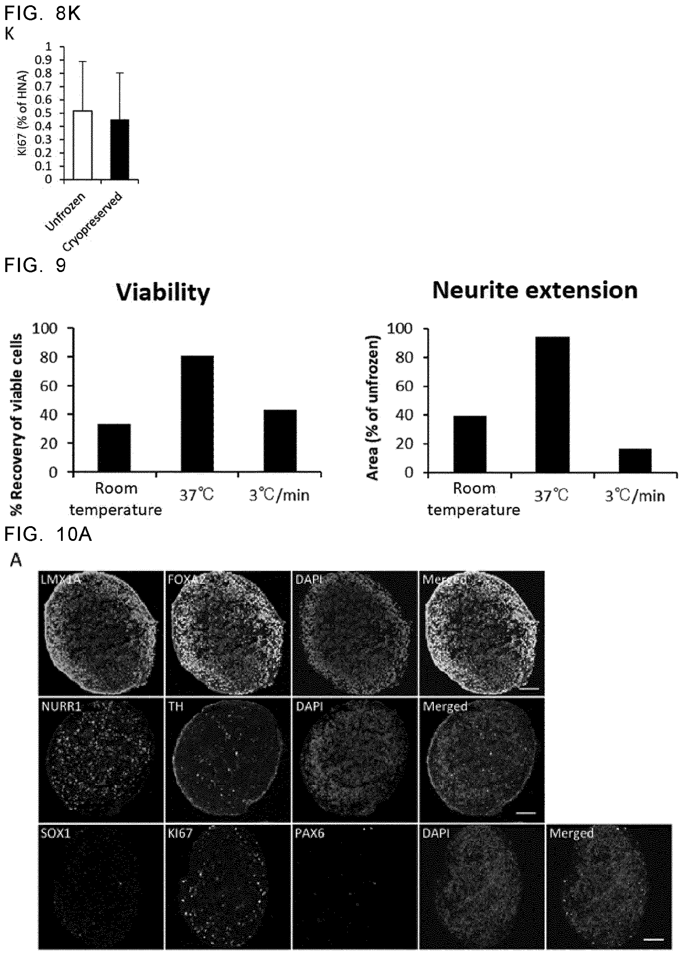

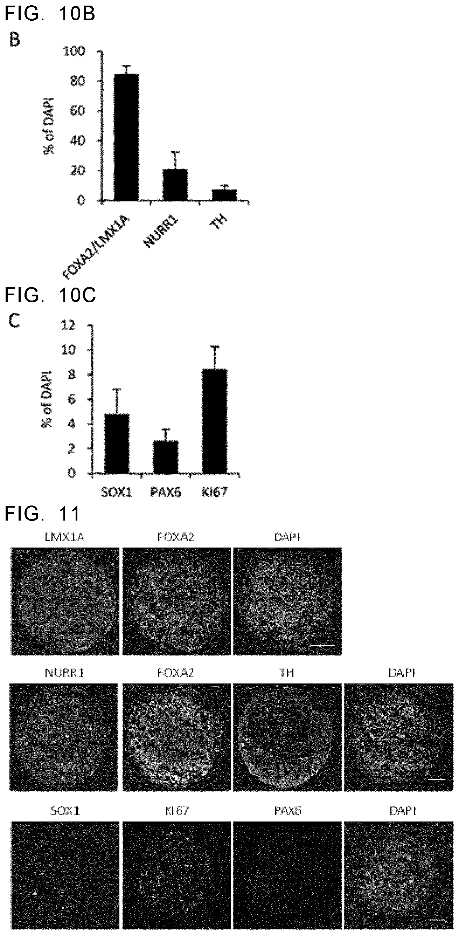

The spheres used above were induced in the same manner as in Figure 1, except that sorting with anti-CORIN antibody was not performed. The results of measuring the expression of the markers in the spheres before freezing are shown in Figure 10 and Table 4.

**[Table 4]**

| | Percentage relative to DAPI-positive cells |
|---|---|
| FOXA2/LMX1A | 8 4. 9 ± 5. 5 % |
| NURR1 | 2 1. 2 ± 1 1. 3 % |
| TH | 7 . 4 ± 2 . 7 % |
| SOX1 | 4. 8 ± 2. 0% |
| PAX6 | 2. 6 ± 1. 0 % |
| KI67 | 8 . 5 ± 1. 8 % |

### (3) Cell number and function of cryopreserved iPSC-derived dopamine-producing neuron progenitor spheres

Based on the above screening results, the properties of the iPSC-derived dopamine-producing neuron progenitor spheres cryopreserved using BBK and the proton freezer were examined after thawing. In order to concentrate the iPSC-derived dopamine-producing neuron progenitor cells to 80% or more, the cells were sorted using CORIN antibody, and subjected to suspension culture under the above-described conditions to form cell aggregates, followed by cryopreservation using BBK and a proton freezer (see Figure 1). The immersion time in BBK was 1 hour.

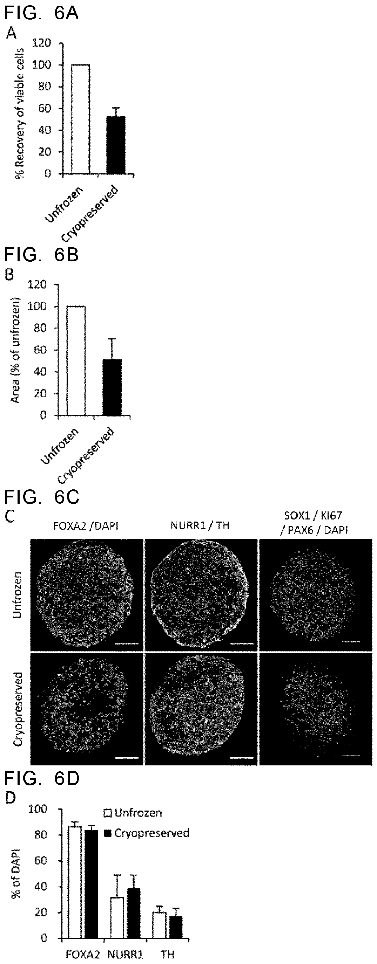

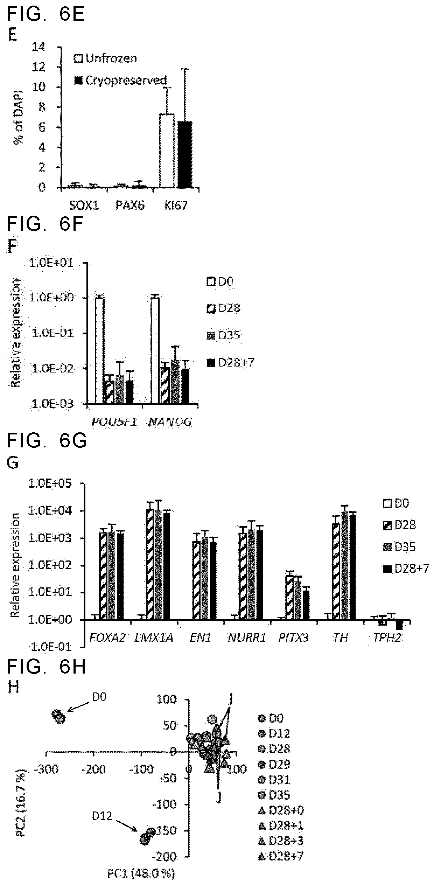

As a result, the cryopreserved spheres were 200 µm to 500 µm in size, and number of cells was 5500 to 12000 (the number of cells was not measured in all spheres and is estimated to be 2000 to 15000 in view of size of the spheres). The resulting spheres showed a cell viability of 52 ± 8% and a neurite extension of 51 ± 19% (Figures 6A, B). To confirm that the spheres are dopamine-producing neuron progenitor cells, protein markers and gene expression were examined 7 days after thawing. As a result of immunocytochemistry, it was found that the cryopreserved spheres expressed almost the same level of the dopamine-producing neuron progenitor cell marker (FOXA2), the dopamine-producing nerve markers (NURR1 and TH), and the proliferating cell marker (KI67) as compared with the unfrozen spheres (Figures 6C to E).

Because the dopamine-producing neuron progenitor cells were highly concentrated by the cell sorting process, cells expressing neural stem cell markers (SOX1 and PAX6) were quite few in both unfrozen and frozen spheres. This indicates that no abnormal proliferation were occurred by the cryopreservation process. Further, a qPCR analysis showed that the expression levels of dopamine-producing neuron progenitor cell markers (FOXA2, LMX1A, and EN1) and dopamine-producing nerve markers (NURR1, PITX3, and TH) were not changed between with or without cryopreservation (Figure 6G). The expression level of pluripotency markers (POU5F1 and NANOG) was maintained less than 1% of day 0 in both spheres (Figure 6F).

In addition, Figure 11 shows the marker expression of the sphere immediately before freezing in the same lot as the sphere shown in Figure 6C, and Table 5 shows the results of measuring the marker expression in multiple lots.

**[Table 5]**

| Lot | 1 | 2 | 3 | 4 | Average | SD |
|---|---|---|---|---|---|---|
| SOX1 | 0.0 | 0.1 | 1.1 | 0.0 | 0.3 | 0.5 |
| PAX6 | 0.0 | 0.0 | 0.0 | 0.5 | 0.1 | 0.3 |
| KI67 | 20.0 | 20.1 | 7.7 | 16.9 | 16.2 | 5.8 |
| NURR1 | 27.8 | 38.9 | 8.5 | 40.8 | 29.0 | 14.8 |
| TH | 13.3 | 12.4 | 9.6 | 12.0 | 11.8 | 1.6 |

Also, the expression rates of FOXA2 and LMX1A were compared (Table 6).

**[Table 6]**

| LOT | 1 | 2 | 3 | 4 | Average | SD |
|---|---|---|---|---|---|---|
| LMX1A | 86.5 | 87.8 | 64.8 | 92.6 | 82.9 | 12.4 |
| FOXA2 | 80.9 | 88.1 | 70.3 | 90.8 | 82.5 | 9.1 |
| LMX1A/FOXA2 | 78.5 | 82.6 | 57.7 | 85.5 | 76.1 | 12.6 |

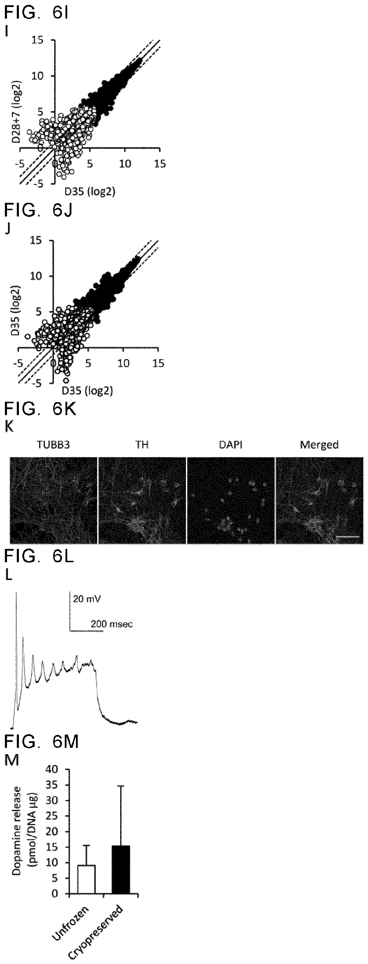

A global PCA analysis using the microarray data was also performed to overview the temporal changes during maturation after the cryopreservation/thawing process. A total of 21,882 probe sets containing significant signals were used for the analysis. The PC1 and PC2 contributions were 48.0% and 15.7%, respectively. PC score plots were clustered into three main groups (day 0 (iPSC), day 12, and after day 28) (Figure 6H). The PCA plots for both unfrozen samples and cryopreserved samples shifted in the positive direction on the PC1 and PC2 axes, regardless of cryopreservation on day 28 and after day 28. Only 206 (0.88%) out of 23349 probe sets showed an increase or decrease more than 2-fold between the unfrozen samples and the cryopreserved samples in the same differentiation batch. In contrast, 222 (0.95%) out of 23275 probe sets showed an increase or decrease more than 2-fold between the unfrozen samples in different batches (Figures 6I, J). This result strongly indicates that the cryopreservation process has not affected the cell properties of the dopamine-producing neuron progenitor cells.

To confirm functional maturation of the iPSC-derived dopamine-producing neuron progenitor spheres, an electrophysiological analysis and a dopamine release measurement were performed. When the cryopreserved spheres were dissociated and cultured on the plate for further maturation, most of the cells gave rise to TH⁺/TUBB3⁺ dopamine-producing nerves on day 28 + 21 (Figure 6K). At this time point, continuous action potentials from the mature dopamine-producing nerves were detected by the whole-cell patch clamp technique, in which current is kept constant (Figure 6L). Dopamine secretion in the unfrozen spheres on day 56 and in the cryopreserved spheres on day 28 + 28 was also detected by LC/MS/MS. The amount of released dopamine was comparable to that of the unfrozen sphere (Figure 6M). Therefore, it was found that cell number and function of the cryopreserved iPSC-derived dopamine-producing neuron progenitor spheres are maintained.

### (4) Engraftment of cryopreserved iPSC-derived dopamine-producing neuron progenitor spheres, and behavior of 6-OHDA lesioned rats

To confirm that the cryopreserved iPSC-derived dopamine-producing neuron progenitor cells could survive after transplantation, the unfrozen sphere and the cryopreserved sphere were transplanted into several types of rat corpus striatum and observed for a short period, followed by determination of a graft survival rate and number of survived cells. The results are shown in Table 7. There was no difference in number of rats with surviving grafts between both of the groups. When the spheres cryopreserved with a proton freezer and BBK were transplanted, the percentage of total surviving cells (HNA⁺) was 42 ± 20% of that of the unfrozen cells.
Table 7. Results of a short-term validation experiment evaluating the graft survival rate of the cryopreserved spheres.

**[Table 7]**

| | Animal | | Duration | Number of animals with cells | | Average number of survived cells | |
|---|---|---|---|---|---|---|---|
| | strain | Model | | Unfrozen | Frozen | Unfrozen | Frozen |
| 1383D6 | Nude | Nomal | 1 month | 3/3 | 3/3 | 25555 | 4554 |
| 1231A3 | Nude | Nomal | 1 month | 2/3 | 2/3 | 13589 | 8330 |
| FF-I01 | SD | 6-OHDA lesioned | 3 months | 2/2 | 1/1 | 31781 | 18326 |
| FF-I01 | Nude | 6-OHDA lesioned | 2 weeks | 3/3 | 3/3 | 31685 | 11520 |

The results of a short-term validation experiment with transplantation indicate that more than twice cryopreserved cells should be transplanted as compared to the unfrozen cells in order to achieve comparable number of survived cells *in vivo.* Therefore, to evaluate number of survived cryopreserved cells and their pharmacological effect, the unfrozen spheres (4 × 10⁵ cells) and the cryopreserved spheres (8 × 10⁵ cells) were transplanted into 6-OHDA lesioned PD disease model rats to measure their methamphetamine-induced rotational movements. Twenty four weeks after the transplantation, abnormal rotations were reduced in both of the groups (Figure 8A) . Immunofluorescent staining at 24 weeks confirmed cell engraftment in all rats that showed behavioral recovery. 55996 ± 3603 HNA⁺ cells were survived in the graft derived from the unfrozen spheres, and 36486 ± 3578 HNA⁺ cells were survived in the graft derived from the cryopreserved spheres (Figures 8B, C). For number of survived HNA⁺ cells, no significant difference was found between both of the groups. Both of the groups had TH⁺ cells survived and showed normal neurite extension (Figures 8D to G). There was no difference in number of TH⁺ cells between the graft derived from the unfrozen spheres and the graft derived from the cryopreserved spheres (3603 ± 1576 cells/graft and 3578 ± 1490 cells/graft) (Figure 8H). TH⁺ cells survived in the grafts derived from the unfrozen spheres and the cryopreserved spheres were 1.1 ± 0.4% and 0.5 ± 0.2% of injected cells, respectively. This result means that about 50% of TH⁺ nerves survived in the graft derived from the cryopreserved spheres compared to that of the unfrozen spheres. Further, the most of the surviving cells in both of the groups were FOXA2⁺ dopamine-producing neuron progenitor cells: 71 ± 6% for the unfrozen spheres and 76 ± 4% for the cryopreserved spheres (Figures 8I, J). In contrast, KI67⁺ proliferating cells, which may cause the risk of abnormal proliferation, were quite few in both of the groups (Figures 8I, K). Therefore, it was found that the cryopreserved iPSC-derived dopamine-producing neuron progenitor spheres are engrafted and improve the behavior of 6-OHDA lesioned rats.

### (5) Expression of dopamine-producing neuron progenitor cell markers

To confirm that the thawed cells have the ability to mature into dopamine-producing neurons similar to the unfrozen cells, protein markers and gene expression were examined over time until 7 days after thawing. As a result of immunocytochemistry, it was found that the cryopreserved spheres continued to express almost the same level of FOXA2 as compared to the unfrozen spheres (Figure 7A). NURR1+ cells increase from day 28 to day 35 in unfrozen cells. The cells after thawing showed the increase of NURR1+ cells at 7 days after thawing, which was slightly later than the unfrozen cells (Figure 7B). Further, a qPCR analysis showed that the expression level of TH was increased in the unfrozen cells with culturing period until day 35. It was confirmed that the expression of TH remained constant in the cells from day 0 to day 3 after thawing, and was increased on day 7 (Figure 7C). Therefore, it was found that the cryopreserved iPSC-derived dopamine-producing neuron progenitor spheres maintain the ability to mature into dopamine-producing neurons.

In addition, Table 8 shows the results of comparing unfrozen cells cultured up to day 35 with the cells frozen on day 28 and cultured for 7 days after thawing in multiple lots.

**[Table 8]**

| LOT | | 1 | 2 | 3 | 4 | Average | SD |
|---|---|---|---|---|---|---|---|
| D35 | SOX1 | 0.5 | 0.0 | 0.0 | 0.3 | 0.2 | 0.2 |
| | PAX6 | 0.0 | 0.0 | 0.4 | 0.2 | 0.1 | 0.2 |
| | KI67 | 10.4 | 6.1 | 4.3 | 8.4 | 7.3 | 2.7 |
| | FOXA2 | 82.9 | 90.4 | 83.0 | 88.8 | 86.3 | 3.9 |
| | NURR1 | 36.8 | 51.3 | 9.5 | 28.5 | 31.5 | 17.5 |
| | TH | 27.0 | 18.4 | 18.8 | 15.8 | 20.0 | 4.9 |
| D28+7 | SOX1 | 0.0 | 0.4 | 0.0 | 0.0 | 0.1 | 0.2 |
| | PAX6 | 0.0 | 0.0 | 0.5 | 0.0 | 0.1 | 0.3 |
| | KI67 | 13.3 | 7.5 | 16.9 | 4.6 | 10.6 | 5.5 |
| | FOXA2 | 83.2 | 88.8 | 81.1 | 82.0 | 83.8 | 3.5 |
| | NURR1 | 44.7 | 41.0 | 23.9 | 46.1 | 38.9 | 10.2 |
| | TH | 15.9 | 9.0 | 23.3 | 20.2 | 17.1 | 6.2 |

### (6) Comparison of thawing conditions (reference)

The thawing conditions for cryopreserved spheres frozen with a proton freezer using BBK as a cryopreservation solution were examined. The tubes stored in the vapor phase of liquid nitrogen were taken out and thawed under three conditions of: (i) room temperature; (ii) a 37°C water bath; and (iii) melting at about 3°C/min (actual value) using a programmed freezer. The times required for thawing were (i) 17 minutes, (ii) 2 minutes, and (iii) about 25 minutes, respectively. The survival rate (recovery of viable cells) and neurite extension activity of the spheres after thawing were measured by the methods described above. As shown in Figure 9, thawing in the 37°C water bath gave the best result.

## Claims

1. A method for freezing a cell aggregate including neural cells and having a three-dimensional structure, which comprises following steps (1) and (2):
(1) contacting the cell aggregate including neural cells and having the three-dimensional structure with a preservation solution at 0°C to 30°C prior to freezing to prepare a preservation solution-soaked cell aggregate; and
(2) cooling the preservation solution-soaked cell aggregate obtained in step (1) from a temperature at least about 5°C higher than the freezing point of the preservation solution to a temperature about 5°C lower than the freezing point at an average cooling speed of 2 to 7 °C/min to freeze the cell aggregate.

2. The method according to claim 1, wherein the average cooling speed in step (2) is 3 to 7 °C/min.

3. The method according to claim 1 or 2, wherein the cell aggregate is contacted with the preservation solution for 15 minutes to 90 minutes, preferably 15 minutes to 60 minutes in step (1).

4. The method according to any one of claims 1 to 3, wherein the preservation solution has the freezing point of from -1°C to -10°C.

5. The method according to any one of claims 1 to 4, wherein the preservation solution is an aqueous solution comprising 7% to 12% of dimethyl sulfoxide and/or propylene glycol, and step (2) is a step of cooling from 0 ± 5°C to -30 ± 5°C at an average cooling speed of 2 to 5 °C/min.

6. The method according to any one of claims 1 to 5, wherein the average cooling speed in step (2) is 3 to 5 °C/min.

7. The method according to any one of claims 1 to 6, wherein the method further comprises a following step (3):
(3) cooling the frozen cell aggregate obtained in step (2) to -50°C or lower.

8. The method according to any one of claims 1 to 7, wherein the cell aggregate including neural cells is a cell aggregate including neural cells derived from pluripotent stem cells.

9. The method according to any one of claims 1 to 8, wherein the cell aggregate including neural cells comprises cells that are positive for at least one of FOXA2, TH, and NURR1.

10. The method according to claim 9, wherein the cell aggregate including neural cells comprises cells positive for FOXA2 and LMX1A.

11. The method according to claim 9, wherein the cell aggregate including neural cells comprises cells positive for FOXA2, TH, and NURR1.

12. The method according to any one of claims 1 to 8, wherein the cell aggregate including neural cells comprises cells positive for FOXA2 and LMX1A in an amount of 40% or more of the total cells and cells positive for TH and NURR1 in an amount of 40% or less of the total cells.

13. The method according to any one of claims 1 to 12, wherein the cell aggregate including neural cells comprises a dopamine-producing neuron progenitor cell and/or a dopamine-producing neuron.

14. The method according to any one of claims 1 to 13, wherein the cell aggregate includes 500 to 150000 cells.

15. The method according to any one of claims 1 to 14, wherein number of cells contained in the preservation solution is 80000 to 5000000 cells/mL, and the cell aggregate has an equivalent spherical diameter of 150 to 1000 µm.

16. The method according to any one of claims 1 to 15, wherein the cell aggregate and the preservation solution have volume of 0.25 mL to 2 mL.

17. The method according to any one of claims 1 to 16, wherein the cell aggregate and the preservation solution are packed in a 0.5 mL to 15 mL container.

18. A method for preserving a cell aggregate including neural cells and having a three-dimensional structure for a long-term, wherein the method comprises holding the frozen cell aggregate obtained by the method according to any one of claims 1 to 17 at or below -80°C.

19. The method according to any one of claims 1 to 18, wherein the obtained frozen cell aggregate can be used without recovery culture after thawing.

20. A composition for transplantation, wherein the composition comprises, as an active ingredient, the cell aggregate frozen or preserved for a long-term by the method according to any one of claims 1 to 19.

21. A frozen composition for transplantation, wherein the composition comprises:
a cell aggregate including 60% or more of dopamine-producing neuron progenitor cells and dopamine-producing neurons derived from a pluripotent stem cell, having an equivalent spherical diameter of 150 µm to 1000 µm and including 500 to 150000 cells; and
a cryopreservation solution comprising 7% to 12% of dimethyl sulfoxide or propylene glycol and having the freezing point of -1°C to -10°C, and
wherein the frozen cell aggregate has following properties:
(1) about 60% or more of the total cells are viable after thawing;
(2) the cells after thawing have a neurite extension activity of 50% or more of that before freezing; and
(3) the rate of the FOXA2-positive cells, LMX1A-positive cells, NURR1-positive cells, and TH-positive cells in the cells viable after thawing are changed within ± 10% from those in the cells before freezing.

22. The composition for transplantation according to claim 20 or 21, wherein number of cells in the composition is 80000 to 5000000 cells/mL, and the composition comprises cells positive for FOXA2 and LMX1A in an amount of 40% or more of the total cells and cells positive for TH and NURR1 in an amount of 40% or less of the total cells.

23. The composition for transplantation according to any one of claims 20 to 22, wherein the cell aggregate has an equivalent spherical diameter of 150 to 1000 µm.

24. The composition for transplantation according to any one of claims 20 to 23, wherein the composition can be used without recovery culture after thawing.

25. The composition for transplantation according to any one of claims 20 to 24, wherein the composition comprises 8 to 192 cell aggregates/mL, the cell aggregate has an equivalent spherical diameter of 150 µm to 1000 µm, and number of cells per container is 80000 to 2400000.

26. The composition for transplantation according to any one of claims 20 to 25, wherein the cell aggregate and the preservation solution have volume of 0.25 mL to 2 mL.

27. The composition for transplantation according to any one of claims 20 to 26, wherein the composition is packed in a 0.5 mL to 15 mL container.

28. A method for producing a composition for transplantation comprising a dopamine-producing neuron progenitor cell as an active ingredient, which comprises:
freezing a cell aggregate by the method according to any one of claims 1 to 17, wherein number of cells in the composition is 80000 to 5000000 cells/mL, the cell aggregate comprises cells positive for FOXA2 and LMX1A in an amount of 40% or more of the total cells and cells positive for TH and NURR1 in an amount of 40% or less of the total cells, and the cell aggregate has an equivalent spherical diameter of 150 µm to 1000 µm.

29. The method for producing the composition for transplantation according to claim 28 containing an aggregate of neural cells as an active ingredient, wherein the composition comprises a population of cell aggregates which have an equivalent spherical diameter of 150 to 1000 µm, number of cells per container is 80000 to 2400000, the cell aggregate and the preservation solution have volume of 0.25 mL to 2 mL, and the composition is packed in a 0.5 mL to 15 mL container.

30. A method for treating a disease requiring regeneration of a dopamine-producing nerve, which comprises following steps of:
(1) thawing the composition for transplantation according to any one of claims 20 to 27 at 30°C to 40°C, preferably at 37°C ± 3°C; and
(2) transplanting the composition for transplantation obtained in (1) into a corpus striatum region of a patient.

31. The method according to claim 30, wherein the cryopreservation solution is replaced with a dosing vehicle without culture after thawing to perform step (2).


SEQUENCE LISTING

<110>  SUMITOMO DAINIPPON PHARMA CO., LTD.
       Kyoto University

<120>  Method of freezing neural cells

<130>  675804

<150>  JP2019-209929
<151>  2019-11-20

<160>  20

<170>  PatentIn version 3.5

<210>  1
<211>  20
<212>  DNA
<213>  Artificial Sequence

<220>
<223>  primer

<400>  1
agaccatctg ccgctttgag                                                   20


<210>  2
<211>  16
<212>  DNA
<213>  Artificial Sequence

<220>
<223>  primer

<400>  2
gcaagggccg cagctt                                                       16


<210>  3
<211>  25
<212>  DNA
<213>  Artificial Sequence

<220>
<223>  primer

<400>  3
ggctctgttt tgctatatcc cctaa                                             25


<210>  4
<211>  24
<212>  DNA
<213>  Artificial Sequence

<220>
<223>  primer

<400>  4
cattacgatg cagcaaatac gaga                                              24

<210>  5
<211>  19
<212>  DNA
<213>  Artificial Sequence

<220>
<223>  primer

<400>  5
ttcaggcccg gctaactct                                                    19


<210>  6
<211>  20
<212>  DNA
<213>  Artificial Sequence

<220>
<223>  primer

<400>  6
agtctcgacc cccacttgct                                                   20


<210>  7
<211>  21
<212>  DNA
<213>  Artificial Sequence

<220>
<223>  primer

<400>  7
gatcccttcc gacagggtct c                                                 21


<210>  8
<211>  20
<212>  DNA
<213>  Artificial Sequence

<220>
<223>  primer

<400>  8
ggtttcccac tctggactgc                                                   20


<210>  9
<211>  23
<212>  DNA
<213>  Artificial Sequence

<220>
<223>  primer

<400>  9
tgggtgtact gcacacgtta ttc                                               23


<210>  10
<211>  21

<212>  DNA
<213>  Artificial Sequence

<220>
<223>  primer

<400>  10
ggaactccgc cttgagtctc t                                                21


<210>  11
<211>  22
<212>  DNA
<213>  Artificial Sequence

<220>
<223>  primer

<400>  11
cgaaaccgaa gagcccacag ga                                               22


<210>  12
<211>  22
<212>  DNA
<213>  Artificial Sequence

<220>
<223>  primer

<400>  12
ggtcatagcc gggttggagt cg                                               22


<210>  13
<211>  22
<212>  DNA
<213>  Artificial Sequence

<220>
<223>  primer

<400>  13
gggccaggag cacagcgact ca                                               22


<210>  14
<211>  22
<212>  DNA
<213>  Artificial Sequence

<220>
<223>  primer

<400>  14
gctgccgccg ctgcttcttt tt                                               22


<210>  15
<211>  20
<212>  DNA
<213>  Artificial Sequence

<220>
<223>  primer

<400>  15
gcagttctcg caggacattg                                                    20


<210>  16
<211>  18
<212>  DNA
<213>  Artificial Sequence

<220>
<223>  primer

<400>  16
cggcaccata ggccttca                                                      18


<210>  17
<211>  21
<212>  DNA
<213>  Artificial Sequence

<220>
<223>  primer

<400>  17
tcagctactt ggcagctcaa c                                                  21


<210>  18
<211>  20
<212>  DNA
<213>  Artificial Sequence

<220>
<223>  primer

<400>  18
cttgccactt tcggtagcag                                                    20


<210>  19
<211>  20
<212>  DNA
<213>  Artificial Sequence

<220>
<223>  primer

<400>  19
ggtcggagtc aacggatttg                                                    20


<210>  20
<211>  20
<212>  DNA
<213>  Artificial Sequence

<220>
<223>  primer

<400> 20
tcagccttga cggtgccatg 20